# EUROPEAN PATENT SPECIFICATION

(11) **EP 4 400 554 B1**
(45) Date of publication and mention of the grant of the patent: **22.07.2026**
(21) Application number: 23874083.1
(22) Date of filing: 08.11.2023
(51) Int. Cl.: C09K 3/18, D06M 13/325, D06M 13/402, C09D 5/00

(54) **REPELLENT AGENT**
REPELLENTMITTEL
AGENT RÉPULSIF

(30) Priority: 09.11.2022 JP 2022179760; 27.06.2023 JP 2023105151
(43) Date of publication of application: 17.07.2024
(73) Proprietor: Daikin Industries, Ltd., Osaka-shi, Osaka 530-0001 (JP)
(72) Inventor: IKEUCHI, Hideyuki, Osaka-shi, Osaka 530-0001 (JP); AKUTA, Ryo, Osaka-shi, Osaka 530-0001 (JP); SHIMANO, Mayumi, Osaka-shi, Osaka 530-0001 (JP); SAKAMAKI, Tatsunori, Osaka-shi, Osaka 530-0001 (JP); MATSUMOTO, Akane, Osaka-shi, Osaka 530-0001 (JP); AIHARA, Marina, Osaka-shi, Osaka 530-0001 (JP); HIGASHI, Masahiro, Osaka-shi, Osaka 530-0001 (JP); KISHIKAWA, Yosuke, Osaka-shi, Osaka 530-0001 (JP)
(74) Representative: Hoffmann Eitle
(86) International application number: PCT/JP2023/040241
(87) International publication number: WO 2024/101396

(56) References cited:
- WO-A1-2019/026593
- WO-A1-2021/118451
- CA-A1- 2 343 267
- CN-A- 1 729 217
- CN-A- 109 722 230
- CN-A- 111 019 736
- GB-A- 1 214 528
- JP-A- 2004 516 248
- JP-A- 2014 098 082
- JP-A- 2017 222 827
- JP-A- 2019 501 915
- JP-A- 2021 075 609
- JP-A- H0 388 850
- JP-A- H01 221 572
- JP-A- H09 507 535
- JP-A- S5 959 998
- JP-A- S59 109 574
- US-A- 2 528 273
- US-A- 3 420 697
- US-A- 3 577 447
- US-A1- 2010 215 799
- US-A1- 2010 233 100
- US-A1- 2014 277 337

## Description

### Technical Field

The present disclosure relates to a repellent.

### Background Art

In recent years, research has progressed on materials and methods that can impart water-repellency, oil-repellency, and the like to various substrates. Patent Literature 1 discloses that a non-fluorinated-based acrylic polymer containing a constituent unit derived from a (meth)acrylic acid ester monomer and a constituent unit derived from silicone oil having a (meth)acryloyl group can be used to impart water-repellency to a fiber substrate. In the invention described in Patent Literature 1, the non-fluorinated-based acrylic polymer is an essential ingredient.
[Patent Literature 2] describes an amphiphilic polymeric material which has a straight or branched chain polymer backbone and a multiplicity of side chains attached to the backbone, wherein the backbone is a copolymer of at least one ethylenically-unsaturated aliphatic hydrocarbon monomer and maleic anhydride, or is a terpolymer of maleic anhydride, ethylene, and a further ethylenically unsaturated monomer.

[Patent Literature 3] describes a chewing gum base including i. 1 to 99% by weight of a polymeric material which has a straight or branched chain carbon-carbon backbone and a multiplicity of side chains, and is substantially insoluble in water; ii. from 0-6% by weight wax; iii. up to 99% by weight of an elastomeric material different to the polymeric material; and iv. 0 to 20% by weight of an elastomer plasticiser; in which the total amount polymeric material and elastomeric material is at least 10% by weight of the chewing gum base.

[Patent Literature 4] describes hydrophobically modified polymers as laundry additives.

[Patent Literature 5] describes a chemical entity comprising more than one chemical components linked to a cellulose binding domain.

[Patent Literature 6] describes an emulsified liquid composition comprising a hydrophobic phase, an emulsifying composition of a protonated amide and a protonated amine having defined carbon to nitrogen atom molar ratios and water.

[Patent Literature 7] describes a method for manufacturing a water repellent composition, water repellent fiber product and water repellent fiber products.

[Patent Literature 8] describes a water repellent composition.

[Patent Literature 9] describes a water repellent composition, a water repellent fiber product and a method for producing water repellent fiber product.

[Patent Literature 10] describes a water-repellent oil-repellent agent composition for an air filter, a method for producing the same and air filter.

[Patent Literature 11] describes a cardable and thermobondable polyolefin-based synthetic fibres treated with hydrophobic spin finishes comprising a cationic antistatic agent and a hydrophobic lubricant, a method for producing the fibres, and nonwoven products prepared from the fibres.

[Patent Literature 12] describes a water- and oil-repellency agents.

[Patent Literature 13] describes a perfluoroalkyl-substituted polyamide compositions prepared by reacting a perfluorocarboxylic acid or derivative thereof with a polyalkylene polyamine.

[Patent Literature 14] describes a water-and oil-repellent and antisoiling finish for Fiber structure.

[Patent Literature 15] describes a water-repellent, oil-repellent, and stain-proofing agent, and more particularly, a water-repellent, oil-repellent, stain-proofing agent composed of a specific urethane compound containing a polyfluoroalkyl group and a specific blender copolymer.

[Patent Literature 16] describes mono-and polyamides of perfluoroalkyl-substituted unsaturated acids.

[Patent Literature 17] describes fluorocarbon derivatives. More particularly, it is concerned with alkanoyl derivatives of fluorinated amides, usable as oil-repellency agents.

[Patent Literature 18] describes sizing of paper by anionic hydrophobic size agent and a cationic holding aid.

[Patent Literature 19] describes a preparation method for an emulsifier for a oil-based drilling fluid, and a drilling fluid.

[Patent Literature 20] describes a sulfur-phosphorus-free organic friction modifier and a preparation method thereof.

[Patent Literature 21] describes a method and a composition for conditioning water and, more particularly, for eliminating foaming conditions in steam boilers.

[Patent Literature 22] describes a medical device, such as a dilatation balloon, including plasticized nylon, and plasticized nylon.

[Patent Literature 23] describes additives for synthetic resins, and more particularly additives which impart high lubricity when added to a rubber-containing styrenic resin and which are low in impact resistance and decrease in heat distortion temperature.

[Patent Literature 24] describes a synthesis method which enables adjustment of introduction rate and introduction position of a fatty acid group to be introduced into an oligoalkylene amine during synthesis of a cationic lipid.

### Citation List

### Patent Literature

[Patent Literature 1] JP 2016-199712 A
[Patent Literature 2] US 2010/0233100
[Patent Literature 3] US 2010/0215799
[Patent Literature 4] CN 1 729 217
[Patent Literature 5] CA 2 343 267
[Patent Literature 6] WO 2021/118451
[Patent Literature 7] JP 2021 075609
[Patent Literature 8] WO 2019/026593
[Patent Literature 9] JP 2017222827
[Patent Literature 10] JP 2014098082
[Patent Literature 11] EP 0739432
[Patent Literature 12] GB 1 214 528
[Patent Literature 13] US 3 420 697
[Patent Literature 14] JP H01221572
[Patent Literature 15] JP S59109574
[Patent Literature 16] US 2002/068802
[Patent Literature 17] US 3 577 447
[Patent Literature 18] JP S5959998
[Patent Literature 19] CN 109722230
[Patent Literature 20] CN 111019736
[Patent Literature 21] US 2 528 273
[Patent Literature 22] US 2014/277337
[Patent Literature 23] JP H0388850
[Patent Literature 24] JP 2019 501915

### Summary of Invention

### Technical Problem

An object of the present disclosure is to provide a use of a repellent for paper that can impart liquid-repellency to paper.

### Solution to Problem

The present disclosure is as described in the attached claims.

### Advantageous Effect of Invention

The repellent of the present disclosure can impart liquid-repellency to paper.

### Description of Embodiments

### <Definition of Terms>

As used herein, the "n valent group" refers to a group having n bonds, i.e., a group forming n bonds. The "n valent organic group" refers to a n valent group containing carbon. Such organic groups are not limited, but can be hydrocarbon groups or derivatives thereof. The derivative of the hydrocarbon group refers to a group that has one or more of N, O, S, Si, amide, sulfonyl, siloxane, carbonyl, carbonyloxy, etc., at an end or in a molecular chain of a hydrocarbon group.

As used herein, the "hydrocarbon group" refers to a group containing carbon and hydrogen and a group in which a hydrogen atom is removed from the hydrocarbon. Such hydrocarbon groups are not limited, but include C₁₋₂₀ hydrocarbon groups, such as an aliphatic hydrocarbon group and an aromatic hydrocarbon group. The above "aliphatic hydrocarbon group" may be either linear, branched, or cyclic, and may be either saturated or unsaturated. The cyclic group may contain a chain structure. The hydrocarbon group may be substituted by one or more substituents.

Whether or not the phrase "independently at each occurrence," "independently with each other", "each independently" or similar expressions are explicitly described herein, unless otherwise described that they are exceptions, when a plurality of terms (symbols) that can occur in a chemical structure is defined, such definition is applied independently to each occurrence.

The chemical structures described herein should be understood not to encompass chemical structures that are recognized by those skilled in the art as being chemically impossible or extremely unstable.

### <Repellent>

The repellent in the present disclosure is such that it imparts liquid-repellency to paper. The repellent of the present disclosure is useful as an additive for paper products, and the paper product obtained from a pulp composition to which the repellent is added can improve its performance (for example, water-resistance, oil-resistance, and paper strength).

The liquid-repellent compound that is an active ingredient in the repellent of the present disclosure is excellent in dispersibility to a liquid medium due to its structure, and the performance of the repellent of the present disclosure can be stabilized.

In the case of using a conventional repellent using a polymer-type compound as an active ingredient, it tends to have a wide molecular weight distribution and contain a relatively large amount of an impurity component. The active ingredient of the repellent of the present application, is, on the other hand, a low molecular weight, and can narrow its molecular weight distribution (singulation), resulting in exhibiting favorable performance.

The repellent in the present disclosure contains a liquid-repellent compound. For example, the repellent in the present disclosure may be a liquid-repellent compound singly. The repellent in the present disclosure may contain other components such as a liquid medium in addition to the liquid-repellent compound.

The repellent in the present disclosure is free of a fluorine compound. Therefore, it is free of any selected from the group consisting of a compound having a fluoroalkyl group having 8 or more carbon atoms, a compound having a perfluoroalkyl group having 8 or more carbon atoms, a compound having a fluoroalkyl group having 4 or more carbon atoms, a compound having a perfluoroalkyl group having 4 or more carbon atoms, a compound having a perfluoroalkyl group, a compound having a fluoroalkyl group and a compound having a fluorine atom. The repellent in the present disclosure can impart liquid-repellency to a substrate even free of these fluorine compounds.

The volume abundance ratio of a particle with a size of 100 µm or larger in the repellent of the present disclosure, as measured by dynamic light scattering or laser diffraction scattering, may be 25% or less, 20% or less, 15% or less, 10% or less, 5% or less, 3% or less, or 1.5% or less, and is preferably 15% or less, or 5% or less. The volume abundance ratio of a particle with a size of 100 µm or larger being in the aforementioned range, is preferable from the viewpoint of coverability. A method for adjusting the volume abundance ratio of a particle with a size of 100 µm or larger, as measured by dynamic light scattering or laser diffraction scattering, to 25% or less, is not limited, but for example, particles in a raw material and/or a dispersion may be micronized by using a pulverizer, a homogenizer or the like.

The volume median diameter of the repellent of the present disclosure as measured by dynamic light scattering or laser diffraction scattering may be 10 nm or larger, 30 nm or larger, 50 nm or larger, 100 nm or larger and preferably 150 nm or larger, or 200 nm or larger, or may be 100 µm or smaller, 90 µm or smaller, 80 µm or smaller, 70 µm or smaller, 60 µm or smaller, 50 µm or smaller, 40 µm or smaller, 30 µm or smaller, 20 µm or smaller, or 10 µm or smaller and it is preferably 30 µm or smaller or 25 µm or smaller. The volume median diameter used herein refers to a median diameter (D50) in a volume-based particle size distribution by dynamic light scattering or laser diffraction scattering.

The repellent in the present disclosure can be suitably utilized as a dispersion (in particular a water dispersion) together with a liquid medium.

### {Liquid-Repellent Compound}

The liquid-repellent compound in the present disclosure can adhere to a substrate and impart liquid-repellency to a substrate.

A HD (n-hexadecane) contact angle of the liquid-repellent compound may be 10° or more, 15° or more, 25° or more, 35° or more, 45° or more, 55° or more, or 65° or more, and may be 100° or less, 90° or less, or 75° or less. The liquid-repellent compound having an HD contact angle of the above lower limit or more can impart favorable liquid-repellency (particularly oil-repellency) to a substrate. The HD contact angle is a static contact angle of the liquid-repellent compound to a spin-coated film, as shown in Example, and is obtained by dropping 2 µL of HD onto the spin-coated film and measuring a contact angle one second after the drop.

A water contact angle of the liquid-repellent compound may be 35° or more, 40° or more, 45° or more, 50° or more, 55° or more, 65° or more, 75° or more, 85° or more, 90° or more, or 100° or more, and may be 160° or less, 140° or less, 130° or less, 120° or less, 110° or less, 100° or less, or 90 or less. The liquid-repellent compound having a water contact angle of the above lower limit or more can impart favorable liquid-repellency (particularly water-repellency) to a substrate. The water contact angle is a static contact angle of the liquid-repellent compound to a spin-coated film, as shown in Example, and is obtained by dropping 2 µL of water onto the spin-coated film and measuring a contact angle one second after the drop.

The liquid-repellent compound is preferably a compound with carbon of biobased origin. A biobased content is measured in accordance with ASTM D6866. The biobased content is preferably 30% or more, more preferably 50% or more, further preferably 60% or more, further preferably 70% or more, and most preferably 80% or more or 90% or more, and for example, it is 100%. The high biobased content refers to less amount of fossil resource-based material used, which is represented by petroleum or the like, and from this perspective, it can be said that the higher the biobased content of the liquid-repellent compound, the more preferable it is.

A melting point of the liquid-repellent compound may be 30°C or higher, 40°C or higher, 60°C or higher, 80°C or higher, 100°C or higher, or 120°C or higher, and is preferably 40°C or higher, and may be 200°C or lower, 150°C or lower, 130°C or lower, 120°C or lower, 110°C or lower, 100°C or lower, 80°C or lower, or 50°C or lower. The liquid-repellent compound may have no melting point.

A biodegradability of the liquid-repellent compound at the time of 180 days is preferably 5% or more of the biodegradability. Because the liquid-repellent compound impacts less on an environmental burden, a higher biodegradability is preferable. The biodegradability of the liquid-repellent compound at the time of 180 days may also be, for example, 10% or more, 20% or more, 30% or more, 50% or more, 60% or more, 70% or more, 80% or more, or 90% or more, and can be preferably 60% or more, more preferably 70% or more, further preferably 80% or more, and most preferably 90% or more. The biodegradability of the liquid-repellent compound at the time of 60 days is preferably 5% or more of the biodegradability. A higher biodegradability is preferable because the liquid-repellent compound impacts less on an environmental burden. The biodegradability of the liquid-repellent compound at the time of 60 days may also be, for example, 10% or more, 15% or more, 20% or more, 25% or more, 30% or more, 35% or more, 40% or more, or 45% or more, and can be preferably 50% or more and more preferably 60% or more. Such a biodegradability can be the biodegradability as specified in JIS K 6953-1 or ASTM D6400.

A molecular weight of the liquid-repellent compound may be 200 or more, 300 or more, 350 or more, 400 or more, 500 or more, 550 or more, or 750 or more, and may be 3,000 or less, 2,500 or less, 2,000 or less, 1,500 or less, 1,000 or less, 900 or less, 800 or less, 750 or less, or 500 or less.

The liquid-repellent compound in the present disclosure is free of any one selected from the group consisting of a fluoroalkyl group having 8 or more carbon atoms, a perfluoroalkyl group having 8 or more carbon atoms, a fluoroalkyl group having 4 or more carbon atoms, a perfluoroalkyl group having 4 or more carbon atoms, a perfluoroalkyl group, a fluoroalkyl group, and a fluorine atom. The liquid-repellent compound can impart liquid-repellency to a substrate, even free of these fluorine-containing groups.

The liquid-repellent compound in the present disclosure may be free of an active hydrogen-containing group. Examples of the active hydrogen-containing group include an amino group (an amino group not adjacent to a carbonyl group, for example, a primary or secondary amino group), a hydroxy group, and a carboxyl group. In particular, the liquid-repellent compound in the present disclosure may be free of a primary or secondary amino group not adjacent to a carbonyl group.

The liquid-repellent compound is a compound
i) represented by the following formula:

   N(-X-Rₙ)ₚ(-H)_{q}-L¹-[N(-X-Rₙ)ᵣ(-H)ₛ-L¹-]ₜ-N(-X-Rₙ)ₚ(-H)_{q}

   wherein
   X is a direct bond or a (1+n)-valent group, composed of one or more selected from the group consisting of a direct bond, -O-, -C(=O)-, -C(=NR'-), -S-, - S(=O)₂-, -NR'-, -C(OR')R'-, -C(OR')(-)₂, -N(-)₂, a divalent to tetravalent aliphatic hydrocarbon group having 1 to 10 carbon atoms, a divalent to tetravalent hydrocarbon aromatic ring, and a divalent to tetravalent heterocyclic ring, wherein
   R' is a hydrogen atom or a hydrocarbon group having 1 to 4 carbon atoms;
   R each independently is a linear or branched monovalent C₆₋₄₀-hydrocarbon group optionally having a substituent;
   L¹ is independently at each occurrence a divalent aliphatic hydrocarbon group or aromatic hydrocarbon group having 2 to 20 carbon atoms optionally interrupted by an oxygen atom and/or a sulfur atom;
   n is independently at each occurrence an integer of 1 or more and 3 or less;
   p is independently at each occurrence an integer of 0 or more and 2 or less;
   q is independently at each occurrence an integer of 0 or more and 2 or less;
   p + q is 2 in each N(-X-Rₙ)ₚ(-H)_{q};
   r is independently at each occurrence 0 or 1;
   s is independently at each occurrence 0 or 1;
   r + s is 1 in each N(-X-Rₙ)ᵣ(-H)ₛ;
   a sum of all p and all r is 1 or more; and
   t is an integer of 0 or more and 10 or less; or
ii) represented by the following formula:

   N(-X-Rₙ)ₚ(-H)_{q}-L²(-X-Rₙ)ᵤ

   wherein
   X is a direct bond or a (1+n)-valent group, composed of one or more selected from the group consisting of a direct bond, -O-, -C(=O)-, -C(=NR'-), -S-, - S(=O)₂-, -NR'-, -C(OR')R'-, -C(OR') (-)₂, -N(-)₂, a divalent to tetravalent aliphatic hydrocarbon group having 1 to 10 carbon atoms, a divalent to tetravalent hydrocarbon aromatic ring, and a divalent to tetravalent heterocyclic ring, wherein R' is a hydrogen atom or a hydrocarbon group having 1 to 4 carbon atoms;
   R each independently is a linear or branched monovalent C₆₋₄₀-hydrocarbon group optionally having a substituent;
   L² is a 1 + u valent aliphatic hydrocarbon group or aromatic hydrocarbon group having 2 to 20 carbon atoms optionally interrupted by an oxygen atom and/or a sulfur atom;
   n is independently at each occurrence an integer of 1 or more and 3 or less;
   p is an integer of 0 or more and 2 or less;
   q is an integer of 0 or more and 2 or less;
   p + q is 2;
   u is an integer of 1 or more and 3 or less;
   a sum of p and u is 1 or more;

### [Amine Backbone]

The liquid-repellent compound in the present disclosure has an amine backbone. The amine backbone has one or more amino groups with a predetermined number of bond (valence), which is obtained by removing a predetermined number of atom or atomic group (for example, hydrogen) from an amine compound. The amino group in the amine backbone refers to a group selected from the group consisting of -NH₂, -NH-, and -N(-)₂, and also includes an amino group adjacent to a carbonyl group contained in an amide group, a urethane group, a urea group, an imide group, or the like. It is to be noted that the amine backbone may be an aliphatic group or an aromatic group, having one or more amino groups, and does not exclude the presence of a heteroatom other than nitrogen.

A molecular weight of the amine backbone may be 30 or more, 50 or more, 100 or more, 200 or more, 300 or more, 400 or more, or 500 or more, and may be 2,800 or less, 2,500 or less, 2,000 or less, 1,500 or less, 1,000 or less, 750 or less, 600 or less, 450 or less, 300 or less, or 250 or less.

The number of carbon atoms of the amine backbone may be 1 or more, 2 or more, 3 or more, 4 or more, 6 or more, 8 or more, 10 or more, 12 or more, 14 or more, 16 or more, or 18 or more, and may be 100 or less, 80 or less, 60 or less, 40 or less, 30 or less, 20 or less, 10 or less, or 5 or less, and it is preferably 50 or less and particularly 30 or less.

The amine backbone has one or more amino groups. The amino group is a monovalent to trivalent amino group and is one or more groups selected from the group consisting of -NH₂, -NH-, and -N(-)₂. The number of the amino group that the amine backbone has may be 1 or more, 2 or more, 3 or more, 4 or more, 5 or more, or 6 or more, and is preferably 2 or more, and it may be 12 or less, 10 or less, 8 or less, 6 or less, 4 or less, 3 or less, 2 or less, or 1.

The amine backbone has a hydrocarbon group (aliphatic hydrocarbon group or aromatic hydrocarbon group). The hydrocarbon group may be cyclic, a branched chain, or linear. The hydrocarbon group may be saturated or unsaturated (for example, saturated). Herein, the hydrocarbon group may be interrupted by an oxygen atom and/or a sulfur atom, or may be composed only of a carbon atom, a nitrogen atom, and a hydrogen atom. For example, the hydrocarbon group may also be a hydrocarbon group (for example, a chain saturated aliphatic hydrocarbon group or an aromatic hydrocarbon group having one or two hydrocarbon aromatic rings) optionally interrupted by an oxygen atom and/or a sulfur atom, or for example, a general hydrocarbon group (for example, a chain saturated aliphatic hydrocarbon group or an aromatic hydrocarbon group having one or two hydrocarbon aromatic rings). When the hydrocarbon group is interrupted by an oxygen atom and/or a sulfur atom, it will have a structure of an ether, thioether, polyether, or polythioether. The number of the hydrocarbon group that the amine backbone has may be 1 or more, 2 or more, 3 or more, 4 or more, 5 or more, or 6 or more, and may be 12 or less, 10 or less, 8 or less, 6 or less, 4 or less, 3 or less, 2 or less, or 1.

The amine backbone may be composed of a monovalent to trivalent amino group and a chain saturated aliphatic hydrocarbon group or aromatic hydrocarbon group optionally interrupted by an oxygen atom and/or a sulfur atom.

A molar ratio of a carbon atom to a nitrogen atom (C/N ratio) in the amine backbone may be 1 or more, 2 or more, 2.5 or more, 3 or more, 3.5 or more, or 4 or more, and may be 8 or less, 7 or less, 6 or less, 5 or less, 4 or less, 3.5 or less, 3 or less, 2.5 or less, or 2 or less, and it is preferably 6 or less or 4 or less.

### (Raw Material Amine Compound)

Examples of the raw material amine compound that is a precursor of the amine backbone include alkylamines such as methylamine, ethylamine, propylamine, butylamine, and dibutylamine; alkylenediamines such as ethylenediamine, propylenediamine, butylenediamine, pentanediamine, hexamethylenediamine, cyclohexanediamine, and methylenebiscyclohexylamine; polyalkylene polyamines such as diethylenetriamine, triethylenetetramine, tris(2-aminoethyl)amine, tetraethylenepentamine, pentaethylenehexamine, dipropylenetriamine, tripropylenetetramine, tris(2-aminopropyl)amine, tetrapropylenepentamine, pentapropylenehexamine, iminobispropylamine, dibutylenetriamine, bis(2-aminoethoxy)ethane, bis(2-aminoethyl)ether, bis[2-(2-aminoethoxy)ethyl]ether, bis[2-(3-aminoprotoxy)ethyl]ether, spermine and spermidine; oxygen- or sulfur-containing aliphatic amines such as 1-aminopropanediol, 2-amino-1,3-propanediol, 3-amino-1,2-propane diol, polyoxypropylene diamine, and polyoxyethylene diamine; aromatic monoamines such as aniline, 1- or 2-naphthylamine, 1-, 2-, or 9-aminoanthracene, 9-aminophenanthracene, and 2-, 3- or 4-aminobiphenyl; monocyclic aromatic polyamines such as o-, m- or p-phenylenediamine, o-, m- or p-xylylenediamine, diaminotoluene, and 2,3-, 2,4- or 2,5-tolylene diamine; polycyclic aromatic polyamines such as diaminobiphenyl, bisaminophenoxyphenylpropane, diaminodiphenyl ether, diaminodiphenyl sulfide, diaminodiphenyl sulfone, diaminobenzophenone, diaminodiphenylmethane, diaminophenylpropane, diamino-phenyl-hexafluoropropane, diaminophenylphenylethane, bisaminophenoxybenzene, bisaminobenzoylbenzene, bisaminodimethylbenzylbenzene, aminophenoxybiphenyl, aminophenoxyphenyl ketone, bisaminoditrifluoromethylbenzylbenzene, aminophenoxyphenyl sulfone, aminophenoxyphenyl ether, aminophenoxyphenylpropane, bis(aminophenoxybenzoyl)benzene, bis(amino phenoxy-α, a-dimethylbenzyl)benzene, bis[(aminoaryloxy)benzoyl]diphenyl ether, bis(amino-α, α-dimethylbenzylphenoxy)benzophenone, aminophenoxyphenyl sulfide, bis[amino-α, α-dimethylbenzylphenoxy]diphenylsulfone, 4,4'-bis[aminophenoxyphenoxy]diphenylsulfone, diaminodiaryloxybenzophenone, diaminoaryloxybenzophenone, 3,3'-dimethoxy-4,4'-diaminobiphenyl, 4,4'-diaminotriphenylmethane, 3,3'-dimethyl-4,4'-diaminobiphenyl, 4,4'-methylenebisaniline, 4,4'-oxydianiline, 1,3-bis(4-aminophenoxy)benzene, 4,4'-diaminodiphenyl ether, and 4,4'-bis(aminophenyl)amine; oxygen- or sulfur-containing polycyclic aromatic polyamines such as 2,2'-bis[4-(4-aminophenoxy)phenyl]propane, 1,3-bis(4-aminophenoxy)benzene, 1,3-bis(3-aminophenoxy)benzene, 3,4'-diaminodiphenyl ether, and 4,4'-diaminodiphenyl sulfide; hydroxyl group-containing polyamines such as 2-hydroxyethylethylenediamine, 2-hydroxyethylpropylenediamine, di-2-hydroxyethylethylenediamine, di-2-hydroxyethylpropylenediamine, 2-hydroxypropylethylenediamine, and di-2-hydroxypropylethylenediamine.

### (Liquid-Repellent Compound Example 1)

Examples of the liquid-repellent compounds include the compound (liquid-repellent compound example 1) represented by the following formula:

N(-X-Rₙ)ₚ(-H)_{q}-L¹-[N(-X-Rₙ)ᵣ(-H)ₛ-L¹-]ₜ-N(-X-Rₙ)ₚ(-H)_{q}

[wherein
X is independently at each occurrence a direct bond or a 1 + n valent group, composed of one or more selected from the group consisting of a direct bond, -O-, -C(=O)-, - C(=NR'-), -S-, -S(=O)₂-, -NR'-, -C(OR')R'-, -C(OR')(-)₂, - N(-)₂, a divalent to tetravalent aliphatic hydrocarbon group having 1 to 10 carbon atoms, a divalent to tetravalent hydrocarbon aromatic ring, and a divalent to tetravalent heterocyclic ring, wherein R' is a hydrogen atom or a hydrocarbon group having 1 to 4 carbon atoms;
R is independently at each occurrence a linear or branched chain monovalent hydrocarbon group having 6 or more and 40 or less carbon atoms and optionally having a substituent; L¹ is independently at each occurrence a divalent aliphatic hydrocarbon group or aromatic hydrocarbon group having 2 to 20 carbon atoms optionally interrupted by an oxygen atom and/or a sulfur atom;
n is independently at each occurrence an integer of 1 or more and 3 or less;
p is independently at each occurrence an integer of 0 or more and 2 or less;
q is independently at each occurrence an integer of 0 or more and 2 or less;
p + q is 2 in each N(-X-Rₙ)ₚ(-H)_{q};
r is independently at each occurrence 0 or 1;
s is independently at each occurrence 0 or 1;
r+s is 1 in each N(-X-Rₙ)ᵣ(-H)ₛ;
the sum of all p and all r is 1 or more; and
t is an integer of 0 or more and 10 or less.].

In the liquid-repellent compound example 1, the details of X, R, and n are incorporated by the above described.

In the liquid-repellent compound example 1, L¹ is a divalent aliphatic hydrocarbon group or aromatic hydrocarbon group having 2 to 20 carbon atoms optionally interrupted by an oxygen atom and/or a sulfur atom, and may be a cyclic, branched chain, or linear hydrocarbon group and is preferably a chain hydrocarbon group or an aromatic hydrocarbon group. L¹ may incorporate the hydrocarbon group described in [Amine Backbone] for use, wherein the hydrocarbon group may be interrupted by an oxygen atom and/or a sulfur atom, or L¹ may be composed only of a carbon atom, a nitrogen atom, and a hydrogen atom. L¹ may be, for example, a saturated or unsaturated (for example, saturated) aliphatic hydrocarbon group or an aromatic hydrocarbon group having 1 to 2 hydrocarbon aromatic rings. L¹ is preferably a cyclic group having both a ring (for example, an aromatic ring) and a chain structure (for example, a linear structure, ether oxygen, or thioether sulfur), and specific examples of L¹ include, for example, a 1,3-phenylenebisalkylene group, a 1,4-phenylenebisalkylene group, a diphenyletherdiyl group, and a diphenylthioetherdiyl group. The number of carbon atom in L¹ may be 2 or more, 3 or more, 4 or more, 6 or more, 8 or more, 10 or more, or 12 or more, and may also be 20 or less, 18 or less, 16 or less, 14 or less, 12 or less, 10 or less, 8 or less, 6 or less, 4 or less, or 3 or less.

In the liquid-repellent compound example 1, p is independently at each occurrence an integer of 0 or more and 2 or less, q is independently at each occurrence an integer of 0 or more and 2 or less, and p + q is 2 in each N(-X-Rₙ)ₚ(-H)_{q}. Preferably, p may be independently at each occurrence 1 or more, and for example, it is 2.

In the liquid-repellent compound example 1, r is independently at each occurrence 0 or 1, s is independently at each occurrence 0 or 1, and r+s is 1 in each N(-X-Rₙ)ᵣ(-H)ₛ. Preferably, p may be independently at each occurrence 1 or more, and for example, it is 2.

The sum of all p and all r is 1 or more, i.e., the liquid-repellent compound example 1 has one or more -X-Rₙ. The sum of all p and all r may be 1 or more, 3 or more, 5 or more, 7 or more, 9 or more, or 12 or more (for example, the sum of all q and all s may be 0), and may be 14 or less, 12 or less, 10 or less, 8 or less, 6 or less, or 4 or less.

In the liquid-repellent compound example 1, t is an integer of 0 or more and 10 or less. t may be 0 or more, 1 or more, 2 or more, 4 or more, or 6 or more, and is preferably 0 or more or 2 or more, and t may be 8 or less, 6 or less, 4 or less, or 3 or less, and for example, it is 0, 1 or 2, for example, 0 or 1.

### (Liquid-Repellent Compound Example 2)

liquid-repellent compound including the liquid-repellent compound example 2 represented by the following formula:

N(-X-Rₙ)ₚ(-H)_{q}-L²(-X-Rₙ)ᵤ

[wherein
X is independently at each occurrence a direct bond or a 1 + n valent group, composed of one or more selected from the group consisting of a direct bond, -O-, -C(=O)-, - C(=NR'-), -S-, -S(=O)₂-, -NR'-, -C(OR')R'-, -C(OR')(-)₂, - N(-)₂, a divalent to tetravalent aliphatic hydrocarbon group having 1 to 10 carbon atoms, a divalent to tetravalent hydrocarbon aromatic ring, and a divalent to tetravalent heterocyclic ring, wherein R' is a hydrogen atom or a hydrocarbon group having 1 to 4 carbon atoms;
R is independently at each occurrence a linear or branched chain monovalent hydrocarbon group having 6 or more and 40 or less carbon atoms and optionally having a substituent; L² is a 1+ u valent aliphatic hydrocarbon group or aromatic hydrocarbon group having 2 to 20 carbon atoms optionally interrupted by an oxygen atom and/or a sulfur atom;
n is independently at each occurrence an integer of 1 or more and 3 or less;
p is an integer of 0 or more and 2 or less;
q is an integer of 0 or more and 2 or less;
p + q is 2;
u is an integer of 1 or more and 3 or less;
the sum of p and u is 1 or more.].

In liquid-repellent compound example 2, the details of X, R, and n are incorporated by the above described.

In the liquid-repellent compound example 2, L² is a 1 + u valent aliphatic hydrocarbon group or aromatic hydrocarbon group having 2 to 20 carbon atoms optionally interrupted by an oxygen atom and/or a sulfur atom, and may be a cyclic, branched chain, or linear hydrocarbon group and is preferably a chain hydrocarbon group or an aromatic hydrocarbon group. L² may incorporate the hydrocarbon group described in [Amine Backbone] for use, wherein the hydrocarbon group may be interrupted by an oxygen atom and/or a sulfur atom, and L² may be composed only of a carbon atom, a nitrogen atom, and a hydrogen atom. L² may be, for example, a saturated or unsaturated (for example, saturated) aliphatic hydrocarbon group or an aromatic hydrocarbon group having 1 to 2 hydrocarbon aromatic rings. L² is preferably a cyclic group having both a ring (for example, an aromatic ring) and a chain structure (for example, a linear structure, ether oxygen, or thioether sulfur), and specific examples of L² include, for example, a 1,3-phenylenebisalkylene group, a 1,4-phenylenebisalkylene group, a diphenyletherdiyl group, and a diphenylthioetherdiyl group. The number of carbon atom in L² may be 2 or more, 3 or more, 4 or more, 6 or more, 8 or more, 10 or more, or 12 or more, and may be 20 or less, 18 or less, 16 or less, 14 or less, 12 or less, 10 or less, 8 or less, 6 or less, 4 or less, or 3 or less.

In the liquid-repellent compound example 2, p is an integer of 0 or more and 2 or less, q is an integer of 0 or more and 2 or less, and p + q is 2. Preferably, p may be 1 or more, and for example it is 2.

In the liquid-repellent compound example 2, u is an integer of 1 or more and 3 or less. u is 1, 2, or 3, for example, 2 or 3.

In the liquid-repellent compound example 2, the sum of p and u is 1 or more, i.e., the liquid-repellent compound example 2 has one or more -X-Rₙ. The sum of all p and u may be 1 or more, 2 or more, 3 or more, 4 or more, or 5 or more (for example, the sum of all q may be 0), and the sum of p and u may be 5 or less, 4 or less, 3 or less, or 2 or less.

### (Specific Example)

Specific examples of the liquid-repellent compounds include the compounds represented by the following formulae: In the following formulae, the details of X, R, and n are incorporated by the above described.

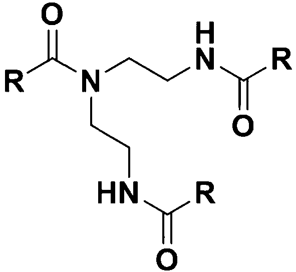

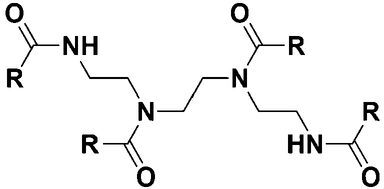

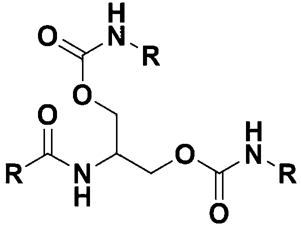

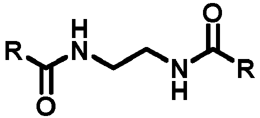

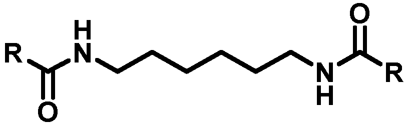

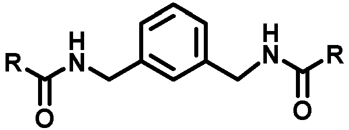

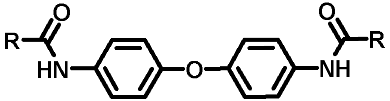

For example, the liquid-repellent compound may be synthetic wax derived from animal or vegetable oil and fat. The synthetic wax may be obtained by condensing a fatty acid derived from animal or vegetable oil and fat and an aliphatic amine or an amine containing an aromatic group. Examples of the synthetic waxes include aliphatic fatty acid amide compounds such as a hydroxy fatty acid amide compound, a palmitic acid amide compound, an octadecanoic acid amide compound, a stearic acid amide compound, an arachidic acid amide compound, a behenic acid amide compound, a lignoceric acid amide compound, an oleic acid amide compound, a linoleic acid amide compound, an α-linolenic acid amide compound, a γ-linolenic acid amide compound, an arachidonic acid amide compound, an icosapentaenoic acid amide compound, and a docosahexaenoic acid amide compound.

### [Production Method of Liquid-Repellent Compound]

The method for producing the liquid-repellent compound is not limited, but examples thereof include, for example, a method for reacting various amines with a R group-containing carboxylic acid in the presence of a condensing agent, if necessary to synthesize the compound; and a method for reacting various amines with a R group-containing acid chloride of carboxylic acid, acid anhydride, isocyanate, or the like to synthesize the compound. The condensing agent may be known condensing agents such as DCC, EDCI, CDI, BOP, COMU, DMT-MM, DPPA, and Py-Bop.

### [Amount of Liquid-Repellent Compound]

The amount of liquid-repellent compound may be, in the repellent, 0.01% by weight or more, 0.5% by weight or more, 1% by weight or more, 3% by weight or more, 5% by weight or more, 10% by weight or more, 20% by weight or more, or 30% by weight or more, and may be 60% by weight or less, 50% by weight or less, 40% by weight or less, 30% by weight or less, 20% by weight or less, 10% by weight or less, 5% by weight or less, or 3% by weight or less.

### {Liquid Medium}

The repellent in the present disclosure may contain a liquid medium. The liquid medium may be water, an organic solvent, or a mixture of water and an organic solvent. The repellent may be a dispersion or solution.

Examples of the organic solvents include esters (for example, esters having 2 to 40 carbon atoms, specifically ethyl acetate and butyl acetate), ketones (for example, ketones having 2 to 40 carbon atoms, specifically methyl ethyl ketone and diisobutyl ketone), alcohols (for example, alcohols having 1 to 40 carbon atoms, specifically isopropyl alcohol), aromatic solvents (for example, toluene and xylene), petroleum-based solvents (for example, alkanes having 5 to 10 carbon atoms, specifically, naphtha and kerosene). The organic solvent is preferably a water-soluble organic solvent. The water-soluble organic solvent may contain compounds having at least one hydroxy group (for example, an alcohol, a polyhydric alcohol such as a glycol-based solvent, and an ether form (for example, a monoether form) of polyhydric alcohol). These may be used singly or in combination of two or more thereof.

### [Amount of Liquid Medium]

The amount of the liquid medium may be 1 part by weight or more, 2 parts by weight or more, 3 parts by weight or more, 4 parts by weight or more, 5 parts by weight or more, 10 parts by weight or more, 20 parts by weight, 30 parts by weight or more, 40 parts by weight or more, 50 parts by weight or more, 100 parts by weight or more, 200 parts by weight or more, 300 parts by weight or more, 500 parts by weight or more, or 1,000 parts by weight or more, relative to 1 part by weight of the liquid-repellent compound, and may be 3,000 parts by weight or less, 2,000 parts by weight or less, 1,000 parts by weight or less, 500 parts by weight or less, 200 parts by weight or less, 175 parts by weight or less, 150 parts by weight or less, 125 parts by weight or less, 100 parts by weight or less, 80 parts by weight or less, 60 parts by weight or less, 40 parts by weight or less, 20 parts by weight or less, or 10 parts by weight or less.

The amount of water may be 1 part by weight or more, 2 parts by weight or more, 3 parts by weight or more, 4 parts by weight or more, 5 parts by weight or more, 10 parts by weight or more, 20 parts by weight or more, 30 parts by weight or more, 40 parts by weight or more, 50 parts by weight or more, 100 parts by weight or more, 200 parts by weight or more, 300 parts by weight or more, 500 parts by weight or more, or 1,000 parts by weight or more, relative to 1 part by weight of the liquid-repellent compound, and may be 3,000 parts by weight parts by weight or less, 2,000 parts by weight or less, 1,000 parts by weight or less, 500 parts by weight or less, 200 parts by weight or less, 175 parts by weight or less, 150 parts by weight or less, 125 parts by weight or less, 100 parts by weight or less, 80 parts by weight or less, 60 parts by weight parts by weight or less, 40 parts by weight or less, 20 parts by weight or less, or 10 parts by weight or less.

The amount of the organic solvent may be 1 part by weight or more, 3 parts by weight or more, 5 parts by weight or more, 10 parts by weight or more, 20 parts by weight or more, 30 parts by weight or more, 40 parts by weight or more, 50 parts by weight or more, 100 parts by weight or more, 200 parts by weight or more, 300 parts by weight or more, 500 parts by weight or more, or 1,000 parts by weight or more, relative to 1 part by weight of the liquid-repellent compound, and may be 3,000 parts by weight or less, 2,000 parts by weight or less, 1,000 parts by weight or less, 500 parts by weight or less, 200 parts by weight or less, 175 parts by weight or less, 150 parts by weight or less, 125 parts by weight or less, 100 parts by weight or less, 80 parts by weight or less, 60 parts by weight or less, 40 parts by weight or less, 20 parts by weight or less, or 10 parts by weight or less.

### {Surfactant}

The repellent may contain a surfactant. The surfactant may contain one or more surfactants selected from a nonionic surfactant, a cationic surfactant, an anionic surfactant, and an amphoteric surfactant. For example, the surfactants may have no fluorine.

### [Nonionic Surfactant]

Examples of the nonionic surfactants include an ether, an ester, an ester ether, an alkanolamide, a polyhydric alcohol and an amine oxide.

The ether is, for example, a compound having an oxyalkylene group (preferably a polyoxyethylene group).

The ester is, for example, an ester of an alcohol and a fatty acid. The alcohol is, for example, an alcohol which is monohydric to hexahydric (particularly dihydric to pentahydric) and has 1 to 50 carbon atoms (particularly 10 to 30 carbon atoms) (for example, an aliphatic alcohol) . Examples of the fatty acids are saturated or unsaturated fatty acids having 2 to 50 carbon atoms, particularly 5 to 30 carbon atoms.

The ester ether is, for example, a compound in which an alkylene oxide (particularly ethylene oxide) is added to an ester of an alcohol and a fatty acid. The alcohol is, for example, an alcohol which is monohydric to hexahydric (particularly dihydric to pentahydric) and has 1 to 50 carbon atoms (particularly 3 to 30 carbon atoms) (for example, an aliphatic alcohol). Examples of the fatty acids are saturated or unsaturated fatty acids having 2 to 50 carbon atoms, particularly 5 to 30 carbon atoms.

The alkanolamide is formed of for example, a fatty acid and an alkanolamine. The alkanolamide may be a monoalkanolamide or a dialkanolamide. Examples of the fatty acids are saturated or unsaturated fatty acids having 2 to 50 carbon atoms, particularly 5 to 30 carbon atoms. The alkanolamine may be an alkanol with 1 to 3 amino groups and 1 to 5 hydroxyl groups, having 2 to 50, particularly 5 to 30 carbon atoms.

The polyhydric alcohol may be, for example, a dihydric to pentahydric alcohol having 10 to 30 carbon atoms.

The amine oxide may be an oxide (for example, having 5 to 50 carbon atoms) of an amine (secondary amine or preferably tertiary amine).

The nonionic surfactant is preferably a nonionic surfactant having an oxyalkylene group (preferably a polyoxyethylene group). The alkylene group in the oxyalkylene group preferably has 2 to 10 carbon atoms. The number of oxyalkylene groups in the molecule of the nonionic surfactant is generally preferably 2 to 100.

The nonionic surfactant is selected from the group consisting of an ether, an ester, an ester ether, an alkanolamide, a polyhydric alcohol, or an amine oxide, and is preferably a nonionic surfactant having an oxyalkylene group.

The nonionic surfactant may be, for example, an alkylene oxide adduct of a linear and/or branched aliphatic (saturated and/or unsaturated) group, a polyalkylene glycol ester of a linear and/or branched fatty acid (saturated and/or unsaturated), a sorbitan ester of a linear and/or branched fatty acid (saturated and/or unsaturated), a glycerin ester of a linear and/or branched fatty acid (saturated and/or unsaturated), a polyglycerol ester of a linear and/or branched fatty acid (saturated and/or unsaturated), a sucrose ester of a linear and/or branched fatty acid (saturated and/or unsaturated), a polyoxyethylene (POE)/polyoxypropylene (POP) copolymer (random copolymer or block copolymer), and an alkylene oxide adduct of acetylene glycol. Among them, the nonionic surfactant is preferably a surfactant such that the structures of the alkylene oxide addition moiety and polyalkylene glycol moiety are polyoxyethylene (POE) or polyoxypropylene (POP) or POE/POP copolymer (which may be a random or block copolymer, for example.).

The nonionic surfactants preferably has a structure free of an aromatic group due to environmental issues (biodegradability, environmental hormones, and the like).

The nonionic surfactant may be the compound represented by the formula:

R¹O-(CH₂CH₂O)ₚ-(R²O)_{q}-R³

[wherein R¹ is an alkyl group having 1 to 22 carbon atoms, an alkenyl group or an acyl group, having 2 to 22 carbon atoms,
R² is each independently the same or different and is an alkylene group having 3 or more carbon atoms (for example, 3 to 10),
R³ is a hydrogen atom, an alkyl group having 1 to 22 carbon atoms, or an alkenyl group having 2 to 22 carbon atoms,
p is a numeral of 2 or more,
q is 0 or a numeral of 1 or more.].

R¹ preferably has 8 to 20 carbon atoms, particularly 10 to 18 carbon atoms. Preferred specific examples of R¹ include an octyl group, a nonyl group, a trimethylnonyl group, a lauryl group, a tridecyl group, an oleyl group, and a stearyl group.

R² is, for example, a propylene group and a butylene group.

In the nonionic surfactant, for example, p may be a numeral of 3 or more (for example, 5 to 200) and q may be a numeral of 2 or more (for example, 5 to 200). Namely, - (R²O)_{q}- may form, for example, a polyoxyalkylene chain.

The nonionic surfactant may be, for example, a polyoxyethylene alkylene alkyl ether comprising a hydrophilic polyoxyethylene chain and a hydrophobic oxyalkylene chain (particularly a polyoxyalkylene chain) in the center. The hydrophobic oxyalkylene chain includes, for example, an oxypropylene chain, an oxybutylene chain, and a styrene chain. The oxypropylene chain is preferred among them.

Specific examples of the nonionic surfactants include a condensation product of ethylene oxide with hexylphenol, isooctatylphenol, hexadecanol, oleic acid, an alkane (C₁₂-C₁₆) thiol, a sorbitan monofatty acid (C₇-C₁₉), an alkyl (C₁₂-C₁₈) amine, or the like, or a sorbitan fatty acid ester, a glycerin fatty acid ester, a sucrose fatty acid ester, a polyoxyethylene alkyl ether, a polyoxyethylene polyoxypropylene alkyl ether, a polyoxyethylene glycerin fatty acid ester, a polyoxyethylene sorbitan fatty acid ester, a propylene glycol fatty acid ester, and a lecithin derivative.

The proportion of the polyoxyethylene block can be 5 to 80% by weight, for example, 30 to 75% by weight, particularly 40 to 70% by weight, based on a molecular weight of the nonionic surfactant (copolymer).

The average molecular weight of the nonionic surfactant is generally 300 to 5,000, for example, 500 to 3,000.

For example, the nonionic surfactant may be used singly or in admixture of two or more. The nonionic surfactant may be a mixture of a compound with an HLB (hydrophilic-hydrophobic balance) of less than 15 (particularly 5 or less) and a compound with an HLB of 15 or more.

### [Cationic Surfactant]

The cationic surfactant is preferably a compound free of an amide group.

The cationic surfactant may be an amine salt, quaternary ammonium salt, or oxyethylene-added ammonium salt. Specific examples of the cationic surfactant are not limited, but include amine salt type surfactants such as an alkylamine salt, aminoalcohol fatty acid derivative, polyamine fatty acid derivative, and imidazoline; quaternary ammonium salt type surfactants such as an alkyltrimethylammonium salt, dialkyldimethylammonium salt, alkyldimethylbenzylammonium salt, pyridinium salt, alkylisoquinolinium salt, benzethonium chloride, and benzalkonium chloride.

Preferred examples of the cationic surfactant are the compound represented by the formula:

R²¹-N⁺(-R²²)(-R²³)(-R²⁴)X⁻

[wherein R²¹, R²², R²³, R²⁴ are each a hydrocarbon group having 1 to 40 carbon atoms, and
X is an anionic group.].

Specific examples of R²¹, R²², R²³, R²⁴ are alkyl groups (for example, a methyl group, a butyl group, a stearyl group, and a palmityl group). Specific examples of X are halogen (for example, chlorine) and acids (hydrochloric acid and acetic acid).

The cationic surfactant is particularly preferably a monoalkyltrimethylammonium salt (with an alkyl having 4 to 40 carbon atoms).

The cationic surfactant is preferably an ammonium salt. The cationic surfactant may be, for example, an ammonium salt represented by the formula:

R¹ₚ-N⁺R²_{q}X⁻

[wherein R¹ is a C12 or more (for example C₁₂ to C₅₀) linear and/or branched aliphatic (saturated and/or unsaturated) group,
R² is H or a C1 to C4 alkyl group, a benzyl group, a polyoxyethylene group (the number of oxyethylene group is, for example, 1 (particularly 2, especially 3) to 50),
and is particularly preferably CH₃ and C₂H₅,
X is a halogen atom (for example) and a C₁ to C₄ fatty acid base,
p is 1 or 2, q is 2 or 3, and p + q=4.].
The number of carbon atoms of R¹ may be 12 to 50, for example, 12 to 30.

Specific examples of the cationic surfactants include dodecyltrimethylammonium acetate, trimethyltetradecylammonium chloride, hexadecyltrimethylammonium bromide, trimethyloctadecylammonium chloride, (dodecylmethylbenzyl)trimethylammonium chloride, benzyldodecyldimethylammonium chloride, methyldodecyl di(hydropolyoxyethylene) ammonium chloride, benzyldodecyl di(hydropolyoxyethylene) ammonium chloride, and N-[2-(diethylamino)ethyl]oleamide hydrochloride.

### [Anionic Surfactant]

Examples of the anionic surfactant include an alkyl ether sulfate, an alkyl sulfate, an alkenyl ether sulfate, an alkenyl sulfate, an olefin sulfonate, an alkanesulfonate, a saturated or unsaturated fatty acid salt, an alkyl or alkenyl ether carbonate, an α-sulfone fatty acid salt, a N-acylamino acid surfactant, a phosphate mono- or diester surfactant, and a sulfosuccinic acid ester.

### [Ampholytic Surfactant]

Examples of the amphoteric surfactants include, for example, alanines, imidazolinium betaines, amidobetaines, and acetic acid betaine, and specific examples of the amphoteric surfactants include, for example, lauryl betaine, stearyl betaine, lauryl carboxymethyl hydroxyethyl imidazolinium betaine, lauryl dimethylamino acetic acid betaine, and fatty acid amidopropyldimethylaminoacetic acid betaine.

The surfactant may be one type or in combination of two or more of nonionic surfactants, cationic surfactants, and amphoteric surfactants, respectively.

### [Amount of Surfactant]

The amount of surfactant may be 0.01 parts by weight or more, 0.1 parts by weight or more, 1 part by weight or more, 3 parts by weight or more, 5 parts by weight or more, 10 parts by weight, 15 parts by weight or more, 20 parts by weight or more, 50 parts by weight or more, 75 parts by weight or more, or 100 parts by weight or more, relative to 100 parts by weight of the liquid-repellent compound, and may be 500 parts by weight or less, 300 parts by weight or less, 200 parts by weight or less, 100 parts by weight or less 30 parts by weight or less, 20 parts by weight or less, 10 parts by weight or less, 5 parts by weight or less, 3 parts by weight or less, or 1 part by weight or less.

### {Silicone }

The repellent in the present disclosure may include silicone (polyorganosiloxane). Containing the silicone enables providing favorable texture and durability in addition to favorable liquid-repellency.

As the silicone, a known silicone can be used, and examples of the silicone include a polydimethylsiloxane and modified silicones (for example, amino-modified silicone, epoxy-modified silicone, carboxy-modified silicone, and methylhydrogen silicone). For example, the silicone may be silicone wax having waxy properties. These may be used singly or in combination of two or more thereof.

A weight-average molecular weight of the silicone may be 1,000 or more, 10,000 or more, or 50,000 or more, and may be 500,000 or less, 250,000 or less, 100,000 or less, or 50,000 or less.

### [Amount of Silicone]

The amount of silicone is 0.1 parts by weight or more, 1 part by weight or more, 3 parts by weight or more, 5 parts by weight or more, 10 parts by weight or more, 15 parts by weight or more, 20 parts by weight or more, 50 parts by weight or more, 75 parts by weight or more, or 100 parts by weight or more, relative to 100 parts by weight of the liquid-repellent compound, and may be 500 parts by weight or less, 300 parts by weight or less, 200 parts by weight or less, 100 parts by weight or less, 50 parts by weight or less, 40 parts by weight or less, 30 parts by weight or less, 20 parts by weight or less, 10 parts by weight or less, or 5 parts by weight or less.

### {Wax}

The repellent in the present disclosure may include wax. Containing the wax can impart favorable liquid-repellency to a substrate.

Examples of the wax include paraffin wax, microcrystalline wax, Fischer-Tropsch wax, polyolefin wax (for example, polyethylene wax and polypropylene wax), oxidized polyolefin wax, silicone wax, animal and vegetable wax, and mineral wax. The paraffin wax is preferred. Specific examples of compounds constituting the wax include normal alkanes (for example, tricosane, tetracosane, pentacosane, hexacosane, heptacosane, octacosane, nonacosane, triacontane, hentriacontane, dotriacontane, tritriacontane, tetratriacontane, pentatriacontane, and hexatriacontane), normal alkenes (for example, 1-eicosene, 1-docosene, 1-tricosene, 1-tetracosene, 1-pentacosene, 1-hexacosene, 1-heptacosene, 1-octacosene, nonacosene, triacontene, hentriacontene, dotriacontene, tritriacontene, tetratriacontene, pentatriacontene, and hexatriacontene). The number of carbon atom in the compound constituting the wax is preferably 20 to 60, for example 25 to 45. A molecular weight of the wax may be 200 to 2,000, for example, 250 to 1,500 or 300 to 1,000. These may be used singly or in combination of two or more thereof.

A melting point of the wax may be 50°C or higher, 55°C or higher, 60°C or higher, 65°C or higher, or 70°C or higher, and is preferably 55°C or higher and more preferably 60°C or higher. The melting point of wax is measured in accordance with JIS K 2235-1991.

### [Amount of Wax]

The amount of wax may be 0.1 parts by weight or more, 1 part by weight or more, 3 parts by weight or more, 5 parts by weight or more, 10 parts by weight or more, 15 parts by weight or more, 20 parts by weight or more, 50 parts by weight or more, 75 parts by weight or more, or 100 parts by weight or more, relative to 100 parts by weight of the liquid-repellent compound, and may be 500 parts by weight or less, 300 parts by weight or less, 200 parts by weight or less, 100 parts by weight or less, 50 parts by weight or less, 40 parts by weight or less, 30 parts by weight or less, 20 parts by weight or less, 10 parts by weight or less, or 5 parts by weight or less.

### [Organic Acid]

The repellent may contain an organic acid. As the organic acid, a known organic acid can be used. Examples of the organic acid preferably include, for example, a carboxylic acid, a sulfonic acid, and a sulfinic acid, with the carboxylic acid being particularly preferred. Examples of the carboxylic acid include, for example, formic acid, acetic acid, propionic acid, butyric acid, oxalic acid, succinic acid, glutaric acid, adipic acid, malic acid, and citric acid, with the formic acid or acetic acid being particularly preferred. In the present disclosure, one type of organic acid may be used, or two or more thereof may be combined for use. For example, formic acid and acetic acid may be combined for use.

### [Amount of Organic Acid]

The amount of organic acid may be 0.1 parts by weight or more, 1 part by weight or more, 3 parts by weight or more, 5 parts by weight or more, 10 parts by weight or more, 15 parts by weight or more, 20 parts by weight or more, 50 parts by weight or more, 75 parts by weight or more, or 100 parts by weight or more, relative to 100 parts by weight of the liquid-repellent compound, and may be 500 parts by weight or less, 300 parts by weight or less, 200 parts by weight or less, 100 parts by weight or less, 50 parts by weight or less, 40 parts by weight or less, 30 parts by weight or less, 20 parts by weight or less, 10 parts by weight or less, or 5 parts by weight or less. The amount of organic acid may be adjusted so that a pH of the repellent is 3 to 10, for example 5 to 9, particularly 6 to 8. For example, the repellent may be acidic (pH of 7 or less, for example 6 or less).

### [Curing Agent]

The repellent may contain a curing agent (active hydrogen-reactive compound or active hydrogen-containing compound).

The curing agent (cross-linking agent) in the repellent can effectively cure the liquid-repellent compound. The curing agent may be an active hydrogen-reactive compound or an active hydrogen-containing compound, which reacts with an active hydrogen or an active hydrogen-reactive group that the liquid-repellent compound has. Examples of the active hydrogen-reactive compound include an isocyanate compound, epoxy compound, chloromethyl group-containing compound, carboxyl group-containing compound, and hydrazide compound. Examples of the active hydrogen-containing compound include a hydroxyl group-containing compound, an amino group-containing compound and a carboxyl group-containing compound, a ketone group-containing compound, a hydrazide compound, and a melamine compound.

The curing agent may contain an isocyanate compound. The isocyanate compound may be a polyisocyanate compound. The polyisocyanate compound is a compound having two or more isocyanate groups in one molecule. The polyisocyanate compound serves as a cross-linking agent. Examples of the polyisocyanate compound include, for example, an aliphatic polyisocyanate, an alicyclic polyisocyanate, an araliphatic polyisocyanate, an aromatic polyisocyanate, and derivatives of these polyisocyanates. The isocyanate compound may be a blocked isocyanate compound (for example, a blocked polyisocyanate compound). The blocked isocyanate compound is a compound in which an isocyanate group of an isocyanate compound is masked with a blocking agent to inhibit reaction.

Examples of the aliphatic polyisocyanates are aliphatic triisocyanates such as trimethylene diisocyanate, tetramethylene diisocyanate, hexamethylene diisocyanate, pentamethylene diisocyanate, 1,2-propylene diisocyanate, 1,2-butylene diisocyanate, 2,3-butylene diisocyanate, 1,3-butylene diisocyanate, 2,4,4- or 2,2,4-trimethylhexamethylene diisocyanate, an aliphatic diisocyanate of 2,6-diisocyanatomethylcaproate, and aliphatic triisocyanates such as lysine ester triisocyanate, 1,4,8-triisocyanateoctane, 1,6,11-triisocyanatoundecane, 1,8-diisocyanato-4-isocyanatomethyloctane, 1,3,6-triisocyanatohexane, 2,5,7-trimethyl-1,8-diisocyanato-5-isocyanatomethyloctane. These may be used singly or in combination of two or more thereof.

Examples of the alicyclic polyisocyanates include, for example, an alicyclic diisocyanate and an alicyclic triisocyanate. Specific examples of the alicyclic polyisocyanate include 1,3-cyclopentene diisocyanate, 3-isocyanatomethyl-3,5,5-trimethylcyclohexyl isocyanate (isophorone diisocyanate), and 1,3,5-triisocyanatocyclohexane. These may be used singly or in combination of two or more thereof.

Examples of the aromatic-aliphatic polyisocyanate include an aromatic-aliphatic diisocyanate and aromatic-aliphatic triisocyanate. Specific examples of the araliphatic polyisocyanate include 1,3- or 1,4-xylylene diisocyanate or a mixture thereof, 1,3- or 1,4-bis(1-isocyanato-1-methylethyl)benzene (tetramethyl xylylene diisocyanate) or a mixture thereof, and 1,3,5-triisocyanatomethylbenzene. These may be used singly or in combination of two or more thereof.

Examples of the aromatic polyisocyanates include an aromatic diisocyanate, aromatic triisocyanate, and aromatic tetraisocyanate. Specific examples of the aromatic polyisocyanate include, for example, m-phenylene diisocyanate, p-phenylene diisocyanate, 4,4'-diphenyl diisocyanate, 1,5-naphthalene diisocyanate, 2,4'- or 4,4'-diphenylmethane diisocyanate, or a mixture thereof, 2,4- or 2,6-tolylene diisocyanate or a mixture thereof, triphenylmethane-4,4',4''-triisocyanate, and 4,4'-diphenylmethane-2,2',5,5'-tetraisocyanate. These may be used singly or in combination of two or more thereof.

Examples of the derivative of the polyisocyanate include various derivatives such as a dimer, trimer, biuret, allophanate, carbodiimide, urethodione, urethoimine, isocyanurate, and iminooxadiazinedione of the aforementioned polyisocyanate compounds. These may be used singly or in combination of two or more thereof.

These polyisocyanates can be used singly or in combination of two or more thereof.

As the polyisocyanate compound, a blocked polyisocyanate compound (blocked isocyanate), which is a compound obtained by blocking isocyanate groups of the polyisocyanate compound with a blocking agent, is preferably used. The blocked polyisocyanate compound is preferably used because it is relatively stable even in solution and can be used in the same solution as solution of the repellent.

The blocking agent is an agent that blocks free isocyanate groups. The blocked polyisocyanate compound, for example, can be heated 100°C or higher, for example, 130°C or higher to regenerate isocyanate groups, facilitating a reaction with hydroxyl groups. Examples of the blocking agent include, for example, a phenolic compound, lactam-based compound, aliphatic alcohol-based compound, and oxime-based compound. The polyisocyanate compound may be used singly or in combination of two or more thereof.

The epoxy compound is a compound having an epoxy group. Examples of the epoxy compound include epoxy compounds having a polyoxyalkylene group, such as a polyglycerol polyglycidyl ether and a polypropylene glycol diglycidyl ether; as well as a sorbitol polyglycidyl ether.

The chloromethyl group-containing compound is a compound having a chloromethyl group. Examples of the chloromethyl group-containing compound include, for example, a chloromethyl polystyrene.

The carboxyl group-containing compound is a compound having a carboxyl group. Examples of the carboxyl group-containing compound include, for example, a (poly)acrylic acid, and a (poly)methacrylic acid.

Specific examples of the ketone group-containing compound include, for example, a (poly)diacetone acrylamide, and diacetone alcohol.

Specific examples of the hydrazide compound include, for example, hydrazine, a carbohydrazide, and adipic acid hydrazide.

Specific examples of the melamine compound include, for example, a melamine resin and a methyl etherified melamine resin.

### [Amount of Curing Agent]

The amount of the curing agent may be 0.1 parts by weight or more, 1 part by weight or more, 3 parts by weight or more, 5 parts by weight or more, 10 parts by weight or more, 15 parts by weight or more, 20 parts by weight or more, 50 parts by weight or more, 75 parts by weight or more, or 100 parts by weight or more, relative to 100 parts by weight of the liquid-repellent compound, and may be 500 parts by weight or less, 300 parts by weight or less, 200 parts by weight or less, 100 parts by weight or less, 50 parts by weight or less, 40 parts by weight or less, 30 parts by weight or less, 20 parts by weight or less, 10 parts by weight or less, and 5 parts by weight or less.

### {Other Component}

The repellent may contain a component other than the aforementioned components. Examples of the other components include, for example, polysaccharides, a paper strengthening agent, an agglomerating agent, a yield improver, a coagulant, a binder resin, an anti-slip agent, a sizing agent, a paper strengthening agent, a filler, an antistatic agent, an antisceptic agent, an ultraviolet absorber, an antibacterial agent, a deodorant, and a fragrance. These may be used singly or in combination of two or more thereof.

In addition to the above components, as other components, for example, other water-repellent and/or oil-repellent agents, a dispersant, a texture modifier, a softening agent, a flame retarder, a coating material fixing agent, a wrinkle-resistant agent, a drying rate adjuster, a cross-linking agent, a film formation agent, a compatibilizer, an antifreezing agent, a viscosity adjuster, a ultraviolet absorber, an antioxidant, a pH adjuster, an insect repellent, an antifoaming agent, an anti-shrinkage agent, a laundry wrinkle-resistant agent, a shape retention agent, a drape retention agent, an ironing improving agent, a brightening agent, a whitening agent, fabric softening clay, a migration-proofing agent such as a polyvinylpyrrolidone, a polymer dispersant, a soil release agent, a scum dispersant, a fluorescent brightening agent such as 4,4-bis(2-sulfostyryl)biphenyldisodium (Tinopal CBS-X manufactured by Ciba Specialty Chemicals Plc), a dye fixing agent, an anti-color fading agent such as 1,4-bis(3-aminopropyl)piperazine, a stain removing agent, enzymes such as cellulase, amylase, protease, lipase, and keratinase as fiber surface modifiers, a foam inhibitor, and silk protein powder that can impart texture and functions of silk such as moisture absorption and release properties, and surface modified products or emulsified dispersions thereof (for example, K-50, K-30, K-10, A-705, S-702, L-710, FP series (Idemitsu Petrochemical Co., Ltd.), hydrolyzed silk liquid (Jomo), SILKGEN G Soluble S (ICHIMARU PHARCOS Co., Ltd.)), an antifouling agent (for example, a nonionic polymer compound composed of an alkylene terephthalate and/or an alkylene isophthalate units and a polyoxyalkylene unit (for example, FR627 manufactured by GOO CHEMICAL CO., LTD.), SRC-1 manufactured by Clariant (Japan), K. K.), can be compounded. These may be used singly or in combination of two or more thereof.

### [Polysaccharide]

Examples of polysaccharides include, for example, starch, xanthan gum, karaya gum, welan gum, guar gum, pectin, tamarind gum, carrageenan, chitosan, gum arabic, locust bean gum, cellulose, alginic acid, agar, dextran, and pullulan. The polysaccharide may be a substituted modified polysaccharide, particularly a modified polysaccharide having a hydroxyl group or a cationic group introduced therein.

### [Paper Strengthening Improver, Agglomerating agent, Yield Improver or Coagulant]

Examples of the paper strengthening improver, agglomerating agent, yield improver or coagulant include, for example, a styrenic polymer (styrene/maleic acid polymer, styrene/acrylic acid polymer), a urea-formaldehyde polymer, a polyethyleneimine, a melamine-formaldehyde polymer, a polyamidoamine-epichlorohydrin polymer, a polyacrylamide-based polymer, a polyamine-based polymer, a polydiallyldimethylammonium chloride, a alkylamine·epichlorohydrin condensate, a condensate of alkylene dichloride and polyalkylenepolyamine, a dicyandiamide formalin condensate, a dimethyldiallylammonium chloride polymer, and an olefin/maleic anhydride polymer.

### [Sizing Agent]

Examples of the sizing agent include a cellulose-reactive sizing agent, for example, a rosin-based sizing agent such as rosin-based soap, rosin-based emulsion/a dispersion, a cellulose-reactive sizing agent, for example, emulsion/dispersions of acid anhydrides such as alkyl and alkenyl succinic anhydrides (ASA), an alkenyl and alkyl ketene dimers (AKD) and multimers thereof, and anionic, cationic and amphoteric polymers of ethylenically unsaturated monomers, for example, a styrene and acrylate copolymer.

### [Antistatic Agent]

Examples of the antistatic agent include, for example, cationic antistatic agents having cationic functional groups such as a quaternary ammonium salt, a pyridinium salt, and primary, secondary, and tertiary amino groups; anionic antistatic agents having anionic functional groups such as a sulfonate salt and a sulfate ester salt, a phosphonate and a phosphate ester salt; amphoteric antistatic agents such as an alkyl betaine and a derivative thereof, imidazoline and a derivative thereof, and alanine and a derivative thereof; and nonionic antistatic agents such an amino alcohol and a derivative thereof, glycerin and a derivative thereof, and a polyethylene glycol and a derivative thereof. For example, an ion conductive polymer obtained by polymerizing or copolymerizing a monomer having an ion conductive group of the cationic, anionic, or amphoteric antistatic agent, may be used. These may be used singly or in combination of two or more thereof.

### [Antiseptic Agent]

The antiseptic agent may be used mainly to enhance antisepsis power and bactericidal power to maintain antiseptic during long-term storage. Examples of the antiseptic agent include isothiazolone-based organosulfur compounds, benzisothiazolone-based organosulfur compounds, benzoic acids, and 2-bromo-2-nitro-1,3-propanediol.

### [Ultraviolet Absorber]

The ultraviolet absorber is an agent that has a protection effect against ultraviolet rays, and is a component that absorbs ultraviolet rays, converts them into infrared rays, visible rays, and the like, and emits them. Examples of the ultraviolet absorber include aminobenzoic acid derivatives, salicylic acid derivatives, silicic acid derivatives, benzophenone derivatives, azole-based compounds, and 4-t-butyl-4'-methoxybenzoylmethane.

### [Antibacterial Agent]

The antibacterial agent is a component that exhibits the effect of inhibiting bacteria from growing on fibers and further exhibits the effect of inhibiting generation of unpleasant odors derived from decomposition products of microorganisms. Examples of the antibacterial agents include, for example, cationic antibacterial agents such as a quaternary ammonium salt, bis-(2-pyridylthio-1-oxide) zinc, a polyhexamethylene biguanidine hydrochloride salt, 8-oxyquinoline, and a polylysine.

### [Deodorant]

Examples of the deodorant include cluster dextrin, methyl-β-cyclodextrin, 2-hydroxypropyl-β-cyclodextrin, monoacetyl-β-cyclodextrin, acylamidopropyl dimethylamine oxide, and an aminocarboxylic acid-based metal complex (the zinc complex of trisodium methylglycine diacetate described in WO2012/090580).

### [Amount of Other Component]

Each amount or the total amount of other components may be 0.1 parts by weight or more, 1 part by weight or more, 3 parts by weight or more, 5 parts by weight or more, 10 parts by weight or more, 15 parts by weight or more, 20 parts by weight or more, 50 parts by weight or more, 75 parts by weight or more, or 100 parts by weight or more, relative to 100 parts by weight of the liquid-repellent compound, and may be 500 parts by weight or less, 300 parts by weight or less, 200 parts by weight or less, 100 parts by weight or less, 50 parts by weight or less, 40 parts by weight or less, 30 parts by weight or less, 20 parts by weight or less, 10 parts by weight or less, or 5 parts by weight or less.

### <Pulp Composition>

The pulp compositions in the present disclosure include the liquid-repellent compound and a pulp substrate. The pulp composition in the present disclosure can be excellent in oil resistance and/or water resistance and preferably both.

The pulp composition in the present disclosure is obtained by adding the liquid-repellent compound to a pulp substrate. The pulp composition may be obtained by treating a pulp substrate with a repellent containing the liquid-repellent compound, and herein the amount of repellent added and a composition thereof may be determined so that each component has a desired amount. Each component that can be contained in the repellent may be separately added to the pulp composition as an additive.

The pulp composition in the present disclosure may be free of any selected from the group consisting of a compound with a fluoroalkyl group having 8 or more carbon atoms, a compound with a perfluoroalkyl group having 8 or more carbon atoms, a compound with a fluoroalkyl group having 4 or more carbon atoms, a compound with a perfluoroalkyl group having 4 or more carbon atoms, a compound with a perfluoroalkyl group, a compound with a fluoroalkyl group, and a compound with a fluorine atom. The pulp compositions in the present disclosure can impart liquid repellency to a substrate even free of these fluorine compounds.

A pH of the pulp composition may be 3 to 10, for example, 5 to 9, particularly 6 to 8, and the amount of each component may be adjusted to achieve such a pH.

### {Pulp Substrate}

The pulp composition contains a pulp substrate. The pulp substrate is composed of pulp and the pulp may be, for example, wood pulp, non-wood pulp, and wastepaper pulp.

Examples of the pulp include softwood kraft pulp obtained from the genus Abies, the genus Pinus, and the like, and hardwood kraft pulp obtained from the genus Acacia, the genus Eucalyptus, the genus Beech, the genus Aspen, and the like (for example, poplar). Examples of the softwood kraft pulp include, for example, softwood unbleached kraft pulp (NUKP), softwood bleached pulp (NBKP), softwood semi-bleached kraft pulp (NSBKP), and softwood sulfite pulp. Examples of the hardwood kraft pulp include, for example, broad leaf tree unbleached kraft pulp (LUKP), broad leaf tree bleached kraft pulp (LBKP), broad leaf tree semi-bleached kraft pulp (LSBKP), and broad leaf tree sulfite pulp. The pulp used may be used singly or in combinations thereof. In addition to the kraft pulp which is softwood kraft pulp and hardwood kraft pulp, there is also mechanical pulp such as stone ground pulp (SGP), pressurized stone ground pulp (PGW), refiner ground pulp (RGP), thermoground pulp (TGP), chemiground pulp (CGP), groundwood pulp (GP), and thermomechanical pulp (TMP). Furthermore, the wastepaper pulp include disintegrated wastepaper pulp produced from wastepaper of tea paper, used kraft envelopes, magazines, newspapers, leaflets, office paper, cardboard, white paper, Kent paper, imitation paper, land certificate paper, and the like, disintegrated/deinked wastepaper pulp, or disintegrated/deinked/bleached wastepaper pulp. Examples of the non-wood pulp include pulp derived from bagasse, kenaf, bamboo, linter, cotton, linen, hemp, ramie, straw, esparto, Manila hemp, Xyzal hemp, yellow hemp, flax, ganpi, Oriental paper bush, paper mulberry, bagasse, and the like.

An average fiber length of the pulp is preferably 0.1 mm or longer, more preferably 0.3 mm or longer, and further preferably 0.5 mm or longer from the viewpoint of improvement of oil resistance, and from the viewpoint of ease of manufacture, it is preferably 5.0 mm or shorter, more preferably 4.0 mm or shorter, further preferably 3.0 mm or shorter, particularly preferably 2.0 mm or shorter, and most preferably 1.0 mm or shorter.

### [Form of Pulp Substrate]

The pulp substrate to which the liquid-repellent compound is added may be in the form of pulp singly, pulp slurry, a pulp product, and the like, and specific examples thereof include bleached or unbleached chemical pulp such as kraft pulp and sulfite pulp, pulp such as bleached or unbleached high-yield pulp such as groundwood pulp, mechanical pulp, or thermomechanical pulp; pulp slurry containing the pulp; pulp products such as paper, a paper container, and a pulp molded product.

### [Amount of Pulp Substrate]

The amount of pulp substrate may be 0.1% by weight or more, 0.5% by weight or more, 1% by weight or more, 3% by weight or more, 5% by weight or more, 10% by weight or more, 20% by weight or more, 30% by weight or more, 50% by weight or more, 75% by weight or more, or 90% by weight or more, in the pulp composition, and may be 99% by weight or less, 75% by weight or less, 50% by weight or less, 40% by weight or less, 30% by weight % or less, 20% by weight or less, 10% by weight or less, 5% by weight or less, 4% by weight or less, or 3% by weight or less. For example, when a pulp composition is fabricated by internal addition, the amount of pulp substrate may be 30% by weight or less in the pulp composition, and when the pulp composition is fabricated by external addition, the amount of pulp substrate may be 75% or more by weight in the pulp composition.

The amount of pulp substrate may be 50% by weight or more, 60% by weight or more, 70% by weight or more, 80% by weight or more, 90% by weight or more, 95% by weight or more, or 99% by weight or more, in a pulp composition excluding a liquid medium, and may be 99.9% by weight or less, 95% by weight or less, 90% by weight or less, 85% by weight or less, 75% by weight or less, 65% by weight or less, or 55% by weight or less.

### {Liquid Medium}

The pulp composition may contain a liquid medium. The liquid medium may be water, an organic solvent, or a mixture of water and an organic solvent, and is typically an aqueous medium, particularly water. The liquid medium may include a liquid medium derived from the repellent.

### [Amount of Liquid Medium]

The amount of liquid medium may be 0.1% by weight or more, 0.5% by weight or more, 1% by weight or more, 3% by weight or more, 5% by weight or more, 10% by weight or more, 20% by weight or more, 30% by weight or more, 50% by weight or more, 75% by weight or more, 90% by weight or more, or 95% by weight or more, in the pulp composition, and may also be 99% by weight or less, 75% by weight or less, 50% by weight or less, 40% by weight or less, 30% by weight or less, 20% by weight or less, 10% by weight or less, 5% by weight or less, 4% by weight or less, or 3% by weight or less. Typically, when a pulp composition is fabricated by internal addition, the amount of liquid medium may be 50% by weight or more, particularly 90% by weight or more in the pulp composition, and when the pulp composition is fabricated by external addition, the amount of liquid medium may be 30% by weight or less, particularly 10% by weight or less in the pulp composition.

### {Liquid-Repellent Compound}

The pulp composition contains a liquid-repellent compound. The details on types of the liquid-repellent compound are incorporated by the description of the liquid-repellent compound in <Repellent>.

### [Amount of Liquid-Repellent Compound]

The amount of liquid-repellent compound may be 0.1% by weight or more, 0.3% by weight or more, 0.5% by weight or more, 0.75% by weight or more, 1.0% by weight or more, 2.0% by weight or more, or 3.0% by weight or more, and is preferably 0.5% by weight or more, relative to a pulp substrate, and it may be 25% by weight or less, 20% by weight or less, 15% by weight or less, 10% by weight or less, 7.5% by weight or less, 5.0% by weight or less, 4.0% by weight or less, 3.0% by weight or less, 2.0% by weight or less, 1.0% by weight or less, 0.75% by weight or less, or 0.5% by weight or less, and it is, for example, 15% by weight or less, 5.0% by weight or less, or 3.0% by weight or less.

The liquid-repellent compound may undergo external addition treatment on a surface of a pulp substrate (for example, pulp products such as paper, a paper container and a pulp molded product), and the amount of liquid-repellent compound contained in a coating layer formed by the external addition treatment, may be 0.01 g/m² or more, 0.03 g/m² or more, 0.05 g/m² or more, 0.1 g/m² or more, 0.3 g/m² or more, 0.5 g/m² or more, or 1.0 g/m² or more, and may be 5.0 g/m² or less, 4.0 g/m² or less, 3.0 g/m² or less, 2.0 g/m² or less, 1.0 g/m² or less, 0.5 g/m² or less, 0.3 g/m² or less, or 0.1 g/m² or less.

### {Dispersant}

The pulp composition may contain a dispersant. The details on the types of the dispersant are incorporated by the description of the dispersants in <Repellent>.

### [Amount of Dispersant]

The amount of dispersant may be 0.1% by weight or more, 0.3% by weight or more, 0.5% by weight or more, 0.75% by weight or more, 1.0% by weight or more, 2.0% by weight or more, or 3.0% by weight or more, relative to a pulp substrate, and may be 10% by weight or less, 7.5% by weight or less, 5.0% by weight or less, 4.0% by weight or less, 3.0% by weight or less, 2.0% by weight or less, 1.0% by weight or less, 0.75% by weight or less, or 0.5% by weight or less, ant it is preferably 5.0% by weight or less and more preferably 3.0% by weight or less.

### {Paper Strengthening Agent}

The pulp composition may contain a paper strengthening agent. Examples of the paper strengthening agents include, for example,
polyacrylamide-based paper strengthening agents such as a cationic polyacrylamide, an anionic polyacrylamide, and an amphoteric polyacrylamide;
polysaccharide-based paper strengthening agents such as starch, enzyme-modified starch, thermochemically modified starch, oxidized starch, esterified starch, etherified starch (for example, hydroxyethylated starch), aldehyde-modified starch, cationized starch, starch, xanthan gum, karaya gum, welan gum, guar gum, pectin, tamarind gum, carrageenan, chitosan, Gum arabic, locust bean gum, cellulose, alginic acid, agar, dextran, cellulose, carboxymethyl cellulose, hydroxyethyl cellulose, hydroxypropyl cellulose, chitin nanofibers, cellulose nanofibers and pullulan, modified polysaccharides thereof (for example, modified polysaccharides into which a hydroxyl group or a cationic group has been introduced);
polyamide-based paper strengthening agents such as a polyamide resin, a polyamine resin, a polyamide-polyamine resin, a polyamide-epichlorohydrin resin, a polyamide-polyamine-epichlorohydrin resin, a polyamide-polyurea-formaldehyde resin, and an epoxidized polyamide resin;
urea/melamine-based paper strengthening agents such as a urea resin, a melamine resin, a urea-formaldehyde resin, and a melamine-formaldehyde resin;
polyvinyl alcohol-based paper strengthening agents such as a polyvinyl alcohol, a fully saponified polyvinyl alcohol, a partially saponified polyvinyl alcohol, a carboxyl-modified polyvinyl alcohol, a silanol-modified polyvinyl alcohol, a cation-modified polyvinyl alcohol, and a terminal alkyl-modified polyvinyl alcohol;
a styrene·butadiene copolymer, a polyvinyl acetate, a vinyl chloride-vinyl acetate copolymer, a polyvinyl chloride, a polyvinylidene dichloride, a polyacrylic acid ester, a fatty acid diamide, a polyethyleneimine resin, a ketone aldehyde resin, and the like.

The paper strengthening agent in the present disclosure is preferably the polyacrylamide-based paper strengthening agent, the polysaccharide-based paper strength agent, or the polyamide-based paper strength agent.

### [Amount of Paper Strengthening Agent]

The total amount of the paper strengthening agents may be 0.1% by weight or more, 0.3% by weight or more, 0.5% by weight or more, 0.75% by weight or more, 1.0% by weight or more, 2.0% by weight or more, or 3.0% by weight or more, relative to the pulp, and may be 10% by weight or less, 7.5% by weight or less, 5.0% by weight or less, 4.0% by weight or less, 3.0% by weight or less, 2.0% by weight or less, 1.0% by weight or less, 0.75% by weight or less, or 0.5% by weight or less, and it is preferably 5.0% by weight or less.

The amount of the polyacrylamide-based paper strengthening agent may be 0.1% by weight or more, 0.2% by weight or more, 0.4% by weight or more, 0.6% by weight or more, 0.8% by weight or more, or 1.0% by weight or more, relative to pulp, and is preferably 0.3% by weight or more, and may be 1.1% by weight or less, 0.9% by weight or less, 0.7% by weight or less, 0.5% by weight or less, or 0.3% by weight or less, relative to the pulp.

The amount of the polysaccharide-based paper strengthening agent may be 0.6% by weight or more, 1.0% by weight or more, 1.5% by weight or more, 2.0% by weight or more, 2.5% by weight or more, 3.0% by weight or more, 3.5% by weight or more, 4.0% by weight or more, or 4.5% by weight or more, and is preferably 2.0% by weight or more, relative to pulp, and may 5% by weight or less, 4.7% by weight or less, 4.2% by weight or less, 3.7% by weight or less, 3.2% by weight or less, 2.7% by weight or less, 2.2% by weight or less, or 1.7% by weight or less, relative to the pulp.

The amount of the polyamide-based paper strengthening agent may be 0.3% by weight or more, 0.4% by weight or more, 0.5% by weight or more, 0.6% by weight or more, 0.7% by weight or more, or 0.8 wt% or more, relative to the pulp, and may be 0.9 wt% or less, 0.8 wt% or less, 0.7 wt% or less, 0.6 wt% or less, 0.5 wt% or less, or 0.4% by weight or less.

### {Sizing Agent}

The pulp composition may contain a sizing agent. Examples of the sizing agents include a cationic sizing agent, an anionic sizing agent, a neutral sizing agent, and an amphoteric sizing agent, and include for example, a rosin-based sizing agent (for example, an acidic rosin-based sizing agent and a neutral rosin-based sizing agent), an alkyl ketene dimer, and an alkenyl succinic anhydride.

### [Amount of Sizing Agent]

The amount of sizing agent may be 0.1% by weight or more, 0.2% by weight or more, 0.3% by weight or more, 0.5% by weight or more, 0.75% by weight or more, 1.0% by weight or more, 2.0% by weight or more, or 3.0% by weight or more, relative to pulp, and may be 10% by weight or less, 7.5% by weight or less, 5.0% by weight or less, 4.0% by weight or less, 3.0% by weight or less, 2.0% by weight or less, 1.0% by weight or less, 0.75% by weight or less, or 0.5% by weight or less.

### {Other Additive}

In addition to the above described, the pulp composition may also contain other additives such as concomitant chemicals for paper used for production of pulp products such as a fixing agent (for example, aluminum sulfate), a coagulant/agglomerating agent (for example, a polyamine-based resin), a yield improver (for example, a polyacrylamide-based resin), and organic acids (for example, formic acid and acetic acid), dyes, a slime control agent, and an antifoaming agent.

### [Amount of Other Additive]

The amount of each other additive each may be 0.01% by weight or more, 0.1% by weight or more, 1% by weight or more, 3% by weight or more, 5% by weight or more, relative to a pulp substrate, and may be 30% by weight or less, 20% by weight or less, 10% by weight or less, 5% by weight or less, 3% by weight or less, or 1% by weight or less.

### <Production Method of Treated Textile Product or Paper Product>

A method for producing a product treated with the repellent in the present disclosure includes a treatment step of treating a substrate with the repellent described above.

The "treatment" refers to applying the repellent to a substrate by dipping, spraying, coating, etc. By the treatment, a liquid-repellent compound that is an active ingredient of the repellent, adheres to an inside and/or on a surface of the substrate. Herein, the adhesion may be physical adhesion or chemical adhesion, and, for example, the liquid-repellent compound may be physically or chemically (by reaction) modified onto a hydroxyl group of paper.

### [Substrate]

A substrate treated with the repellent in the present disclosure is a paper product.

Examples of the substrate for paper products include, for example, bleached or unbleached chemical pulp such as kraft pulp or sulfite pulp, bleached or unbleached high-yield pulp such as groundwood pulp, mechanical pulp, or thermomechanical pulp, paper made from wastepaper pulp such as wastepaper of newspapers, magazines, and cardboard, and deinked wastepaper, a container made of paper, and a molded product made of paper. Specific examples of the paper products include, for example, a food packaging material, a food container, gypsum liner board base paper, coated base paper, medium-quality paper, a general liner and core, neutral pure white roll paper, a neutral liner, a rust-proof liner, and metal pasted paper, kraft paper, neutral printing writing paper, neutral coated base paper, neutral PPC paper, neutral thermal paper, neutral pressure-sensitive base paper, neutral inkjet paper and neutral information paper, and molded paper (mold container), and suitable examples thereof include the food packaging material and the food container. Treating a paper substrate with the repellent of the present disclosure enables obtaining, for example, paper products imparted with water-repellency, oil-repellency, oil resistance, and water resistance (water-repellent paper, oil-repellent paper, oil-resistant paper, and water-resistant paper).

A substrate to be treated with the repellent of the present disclosure is not limited to textile products or paper products, and it may also include, for example, stone, a filter (for example, an electrostatic filter), a dust-protective mask, fuel cell parts (for example, a gas diffusion electrode and a gas diffusion support), glass, wood, leather, fur, asbestos, brick, cement, metal and oxide, a ceramic product, a plastic, a painted surface, and a plaster.

When the substrate is glass, a glass product to be produced may be an optical member. A certain layer (or film), such as a hard coat layer or an antireflection layer, may be formed on the surface (outermost layer) of a glass substrate. The antireflection layer may be any of a single-layer antireflection layer and a multi-layer antireflection layer. Examples of inorganic substances usable in the antireflection layer include SiO₂, SiO, ZrO₂, TiO₂, TiO, Ti₂O₃, Ti₂O₅, Al₂O₃, Ta₂O₅, CeO₂, MgO, Y₂O₃, SnO₂, MgF₂, and WO₃. One of these inorganic substances may be used singly, or two or more may be used in combination (e.g., as a mixture). In the case of a multi-layer antireflection layer, SiO₂ and/or SiO is preferably used in the outermost layer thereof. When the article to be produced is an optical glass component for a touch panel, a part of the surface of the substrate (glass) may have a transparent electrode such as a thin film in which indium tin oxide (ITO), indium zinc oxide, or the like is used. The substrate, according to its specific configuration or the like, may have an insulating layer, an adhesive layer, a protecting layer, a decorated frame layer (I-CON), an atomizing film layer, a hard coating layer, a polarizing film, a phase difference film, a liquid crystal display module, or the like.

### [Treatment Method]

The repellent of the present disclosure can be applied to a substrate as a treatment agent (particularly a surface-treating agent) by a conventionally known method. The treatment method may be a method for dispersing the repellent in the present disclosure in an organic solvent or water, if necessary, to dilute it and allowing it to adhere to an inside of a substrate and/or on a surface thereof by a known method such as dip coating, spray coating, and foam coating. After drying, a textile product to which a solid component of the repellent has been adhered, is obtained. If necessary, the repellent of the present disclosure may be applied in combination of a suitable cross-linking agent, and curing may be carried out. If necessary, the repellent of the present disclosure can be further combined for use with various additives such as water- and/or oil-repellent agents, an anti-slip agent, an antistatic agent, a texture modifier, a softening agent, an antibacterial agent, a flame retarder, a coating material fixing agent, a wrinkle-resistant agent, a drying rate adjuster, a cross-linking agent, a film formation agent, a compatibilizer, an antifreezing agent, a viscosity modifier, an ultraviolet absorber, an antioxidant, a pH adjuster, an insect repellent, and an antifoaming agent. Examples of the various additives may be the same as those explained in the section "Other Component" in the water-repellent composition described above. A concentration of the repellent in a treatment agent brought into contact with a substrate may be appropriately changed depending on its use, and may be 0.01 to 10% by weight, for example 0.05 to 5% by weight.

The repellent can be applied to a substrate by any of methods known for treating a substrate with liquid. The substrate may be immersed in the repellent, or solution may be adhered or sprayed onto the substrate. The treated substrate is preferably dried and cured by heating in order to develop liquid-repellency. The heating temperature may be, for example, 100°C to 200°C, 100°C to 170°C, or 100°C to 120°C. Favorable performance can be obtained even by heating at lowered temperatures (for example, 100°C to 140°C) in the present disclosure. In the present disclosure, the heating time may be 5 seconds to 60 minutes, for example, 30 seconds to 3 minutes. When the textile product is paper, the paper may undergo coating, or solution may be adhered or sprayed onto the paper, or the solution may be mixed with pulp slurry before papermaking to undergo treatment. The treatment may be external addition treatment or internal addition treatment. Alternatively, the repellent may be applied to a textile product by a cleaning method, and for example, it may be applied to a textile product in, for example, washing application or a dry cleaning method.

### [Paper Substrate Treatment]

Examples of paper substrates (pulp substrates) include paper, a container made of paper, and a molded product made of paper (for example, a pulp mold).

The paper product (paper) can be produced by a conventionally known papermaking method. An internal addition treatment method in which the repellent is added to pulp slurry before papermaking, or an external addition treatment method in which the repellent is applied to paper after papermaking, can be employed.

The internal addition treatment method may refer to a treatment method for adding the repellent to pulp slurry before papermaking. The internal addition treatment method may include, but is not limited to, one or more of a step of adding the repellent to pulp slurry and stirring and mixing them; a step of sucking and dehydrating the pulp composition prepared in the step through a reticular body of predetermined shape and depositing the pulp composition then to form a pulp mold intermediate; and a step of molding and drying the pulp mold intermediate by using a heated molding mold to obtain paper, a container made of paper, and a molded product made of paper. After having been lightly dried at room temperature or elevated temperature, the treated paper may be arbitrarily subjected to heat treatment, depending on the nature of the paper. Temperature of the heat treatment may be 150°C or higher, 180°C or higher, or 210°C or higher, and may also be 300°C or lower, 250°C or lower, or 200°C or lower, particularly 80°C to 180°C. Carrying out the heat treatment in such a temperature range enables exhibiting, for example, excellent oil resistance and water resistance.

A size press used in an external addition treatment method, can be divided into the following types depending on a coating method.

One coating method involves supplying a coating liquid (size liquid) to a nip portion formed by passing paper between two rubber rolls, creating a pool of the coating liquid called a pond, and allowing the paper to pass through this pool to coat both sides of the paper with the size liquid, which is a method employed for a so-called pound-type two-roll size press. Another coating method is a method used for a gate roll type size press in which a size liquid is applied by a surface transfer type, and a rod metering type size press. In the pound-type two-roll size press, the size liquid easily penetrates into an inside of paper, and in the surface transfer type, a size liquid component is likely to stay on a surface of the paper. In the surface transfer type, a coating layer is likely to stay on a surface of paper more than in the pound-type two-roll size press, and the amount of coating layer formed on the surface is more than in the pound-type two-roll size press. In the present disclosure, even in the case of using the former pound-type two-roll size press, performance can be imparted to paper. After having been lightly dried at room temperature or elevated temperature, the paper treated in such a manner is arbitrarily accompanied by heat treatment that can have a temperature range of up to 300°C, for example up to 200°C, and particularly the temperature range of 80°C to 180°C, depending on the nature of the paper, as a result of which excellent oil resistance, water resistance, and the like can be exhibited.

The present disclosure can be used, for example, in gypsum liner board base paper, coated base paper, medium-quality paper, a general liner and core, neutral pure white roll paper, a neutral liner, a rust-proof liner, and metal pasted paper, and kraft paper. The present disclosure can also be used, for example, in neutral printing writing paper, neutral coated base paper, neutral PPC paper, neutral thermal paper, neutral pressure-sensitive base paper, neutral inkjet paper and neutral information paper.

As pulp raw materials, any of bleached or unbleached chemical pulp such as kraft pulp or sulfite pulp; bleached or unbleached high-yield pulp such as groundwood pulp, mechanical pulp, or thermomechanical pulp; and wastepaper pulp such as wastepaper of newspapers, magazines, and cardboard, or deinked wastepaper, can be used. Moreover, a blend of the aforementioned pulp raw material and a synthetic fibers such as asbestos, a polyamide, a polyimide, a polyester, a polyolefin, or a polyvinyl alcohol, can also be used.

A sizing agent can be added to improve water resistance of paper. Examples of the sizing agent are a cationic sizing agent, an anionic sizing agent, and a rosin-based sizing agent (for example, an acidic rosin-based sizing agent and a neutral rosin-based sizing agent) . The amount of sizing agent may be 0.01 to 5% by weight relative to pulp.

For paper, as chemicals for papermaking used to such an extent that they are usually used, if necessary, paper strengthening agents such as starch, modified starch, carboxymethyl cellulose, and a polyamide polyamine-epichlorohydrin resin, and additives used in production of paper, such as an agglomerating agent, a fixing agent, a yield improver, a dye, a fluorescent dye, a slime control agent, and an antifoaming agent, can be used. Starch and/or modified starch are preferably used. If necessary, paper can be coated with the repellent together with starch, a polyvinyl alcohol, a dye, a coating color, an anti-slip agent, or the like, for example, by a size press, a gate roll coater, a bill blade coater, a calender, or the like.

In external addition, the amount of liquid-repellent compound contained in a coating layer is preferably 0.01 to 2.0 g/m² and particularly 0.1 to 1.0 g/m². The coating layer is preferably formed of the repellent and starch and/or modified starch. The solid content of the repellent for paper in the coating layer is preferably 2 g/m² or less.

In internal addition, the repellent is preferably mixed with pulp so that the amount of repellent is 0.01 to 50 parts by weight or 0.01 to 30 parts by weight, for example 0.01 to 10 parts by weight and particularly 0.2 to 5.0 parts by weight, relative to 100 parts by weight of pulp that forms paper.

In external addition, even when employing a so-called pound type two-roll size press treatment in which a treatment liquid is stored between rolls, and base paper is passed through the treatment liquid between the rolls at an arbitrary roll speed and nip pressure, oil-repellency can be imparted to paper.

In external addition treatment, a paper substrate may contain additives such as a sizing agent, a paper strengthening agent, an agglomerating agent, or yield improver or a coagulant. The additives may be nonionic, cationic, anionic or amphoteric. An ionic charge density of the additive may be -10,000 to 10,000 µeq/g, preferably -4,000 to 8,000 µeq/g, and more preferably -1,000 to 7,000 µeq/g. The additive (solid content or active ingredient) such as a sizing agent, a paper strengthening agent, an agglomerating agent, a yield improver, or a coagulant can be generally used in an amount of 0.1 to 10% by weight (for example, 0.2 to 5.0% by weight) relative to pulp. In the case of a paper substrate containing a cationic additive (for example, a sizing agent, a paper strengthening agent, an agglomerating agent, a yield improver, or a coagulant), the repellent is preferably anionic.

In internal addition treatment, pulp slurry having a pulp concentration of 0.5 to 5.0% by weight (for example, 2.5 to 4.0% by weight) preferably undergo papermaking. The pulp slurry can be added with an additive (for example, a sizing agent, a paper strengthening agent, an agglomerating agent, a yield improver, or a coagulant) and the liquid-repellent compound. Examples of the additive (for example, a sizing agent, a paper strengthening agent, an agglomerating agent, a yield improver, or a coagulant) include an alkyl ketene dimer, an alkenyl succinic anhydride, a styrenic polymer (styrene/maleic acid polymer, styrene/acrylic acid-based polymer), a urea-formaldehyde polymer, a polyethyleneimine, a melamine-formaldehyde polymer, a polyamideamine-epichlorohydrin polymer, a polyacrylamide-based polymer, a polyamine-based polymer, a polydiallyldimethylammonium chloride, an alkylamine·epichlorohydrin condensate, a condensate of alkylene dichloride and polyalkylene polyamine, a dicyandiamide·formalin condensate, a dimethyldiallylammonium chloride polymer, and an olefin/maleic anhydride polymer.

The phrase "treatment" refers to applying a liquid-repellent compound to a treatment target, by dipping, spray application, or the like. The treatment allows an active ingredient of the liquid- and oil-repellent compound to penetrate into the treatment target and/or adhere on a surface of the treatment target.

### Examples

The present disclosure will be described in more detail below by way to Examples, but the present disclosure is not limited to these Examples.

### <Test Method>

The test procedures are as follows.

### [Pulp Mold Fabrication]

A pulp mold was fabricated by using an automatic mold molding machine. In the lower part thereof, a reticular body was arranged on a pulp mold molding mold made of metal with many suction holes, a metal tank was arranged in the upper part, and pulp slurry was charged in the upper metal tank. From the opposite side of the pulp mold molding mold to the side where the reticular body was arranged, a pulp-containing aqueous composition was suctioned and dehydrated through the pulp mold molding mold and the reticular body at 0.1 to 1 MPa by a vacuum pump, and solids (pulp, etc.) contained in the pulp-containing aqueous composition were allowed to deposit on the reticular body to obtain a pulp mold intermediate. The resulting pulp mold intermediate is then dried by applying pressure of 0.1 to 1 MPa thereto from the tops and bottoms of male and female molding molds made of metal, which have been heated to 60 to 200°C. This produces a pulp mold molded into a shape of a container.

### [Practical Oil-Resistance Test at 65°C]

The pulp mold was pretreated by storing it for 12 hours under the conditions of 23°C and 50% humidity. After pouring 100 ml of corn oil at 65°C into the pulp mold and allowing it to stand at room temperature for 45 minutes, the corn oil was removed from the pulp mold and a degree of oil staining in the pulp mold was evaluated. The evaluation scores were set as follows according to the degrees of staining.
5: No stains inside the pulp mold.
4: Stains inside the pulp mold. No stains on the backside.
3: Stains inside the pulp mold. Slight stain bleed out on the backside.
2: Stains inside the pulp mold. The area of stain bleed out to the backside is less than 50% of the total area.
1: Stains inside the pulp mold. The area of stain bleed out to the backside is 50% or more and less than 100% of the total area.
0: Stains over the backside.

### [Practical Oil-Resistance Test at 80°C]

The pulp mold was pretreated by storing it for 12 hours under the conditions of 23°C and 50% humidity. The same operations and evaluations as in [Practical Oil-Resistance Test at 65°C] were performed except that corn oil at 80°C was used instead of the corn oil at 65°C.

### [Practical Oil-Resistance Test at 100°C]

The pulp mold was pretreated by storing it for 12 hours under the conditions of 23°C and 50% humidity. The same operations and evaluations as in [Practical Oil-Resistance Test at 65°C] were performed except that corn oil at 100°C was used instead of the corn oil at 65°C.

### [Practical Oil-Resistance Test at 110°C]

The pulp mold was pretreated by storing it for 12 hours under the conditions of 23°C and 50% humidity. The same operations and evaluations as in [Practical Oil-Resistance Test at 65°C] were performed except that corn oil at 110°C was used instead of the corn oil at 65°C.

### [Practical Oil-Resistance Test at 120°C]

The pulp mold was pretreated by storing it for 12 hours under the conditions of 23°C and 50% humidity. The same operations and evaluations as in [Practical Oil-Resistance Test at 65°C] were performed except that corn oil at 120°C was used instead of the corn oil at 65°C.

### [Practical Water-Resistance Test]

The pulp mold was pretreated by storing it for 12 hours under the conditions of 23°C and 50% humidity. After pouring 100 ml of water at 100°C into the pulp mold and allowing it to stand at room temperature for 30 minutes, the water was removed from the pulp mold and a degree of water staining in the pulp mold was evaluated. The evaluation scores were set as follows according to the degrees of staining.
5: No stains inside the pulp mold.
4: Stains inside the pulp mold. No stains on the backside.
3: Stains inside the pulp mold. Slight stain bleed out to the backside.
2: Stains inside the pulp mold. The area of stain bleed out to the backside is less than 50% of the total area.
1: Stains inside the pulp mold. The area of stain bleed out to the backside is 50% or more and less than 100% of the total area.
0: Stains on the entire backside.

### [Fabrication of Treated Paper]

Thin paper with water resistance (Cobb value) of 52 g/m², a basis weight of 45 g/m², and a density of 0.60 g/m³ was coated three times with solution of a liquid-repellent compound with 14.9 mg/cm³ by using a Baker-type applicator with a gap set to 0 mil, followed by annealed at 140°C for 1 minute to create treated paper. Chloroform was used for solution preparation of the coating solution. In the case of the liquid-repellent compound being insoluble in chloroform, an organic solvent such as toluene or acetone was used.

### [KIT Test (Oil Resistance)]

The oil resistance was measured by a 3M kit test (TAPPI T-559cm-02). In the 3M kit test method, test oil compounded with castor oil, toluene, and heptane is placed on a surface of treated paper, and the test oil was wiped off after 15 seconds, and the treated paper is evaluated for the presence or absence of oil staining. Tests were conducted with test oils from kit numbers 1 to 6, and the largest kit number with no staining observed was used as the evaluation result for oil resistance.

### [Corn Oil-Resistance Test (Oil Resistance)]

Corn oil is placed on a surface of the treated paper, and when the test oil was wiped off after 15 seconds, the treated paper is evaluated for the presence or absence of oil staining.

In the case of no stains observed, it is evaluated as "○ "; and in the case of stains being observed, it is evaluated as "×".

### [HD Contact Angle]

A silicon wafer was coated with solution (or dispersion) of a liquid-repellent compound with a solid concentration of 1.0% under the conditions of 2,500 rpm for 25 seconds to obtain a smooth spin-coated film. This was then heated at 140°C for 1 minute to obtain a compound-treated silicon wafer. Chloroform was used as the solvent or dispersant. 2 µL of HD (hexadecane) was dropped onto the compound-treated silicon wafer, and a static contact angle one second after the drop was taken as the HD contact angle of the liquid-repellent compound.

### [Tensile Strength of Pulp Mold Product]

The tensile strength of the pulp mold was not limited, but was evaluated by the specific tensile strength in a dry state as measured in accordance with JIS P 8113: 2006. A test piece 15 mm wide, 50 mm long, and 0.8 mm thick was cut from the bottom of the pulp mold, and used. An apparatus used was an autograph manufactured by SHIMADZU CORPORATION, and a test was conducted under the conditions of a length of 30 mm of the test piece and a tensile speed of 10 mm/min.

### [Fabric Water-Repellency Test] - reference test

A treatment liquid with a solid concentration of 16.8 mg/mL was prepared by using chloroform as a solvent, and the fabric was immersed in the treatment liquid and then passed through a mangle to evaluate water-repellency using the heat-treated test fabric.

The water-repellency of the treated fabric was evaluated in accordance with the spray method of JIS-L-1092 (AATCC-22). The water-repellency is scored by the water-repellency No. as shown in the table below, which indicates that the higher the score, the better the water-repellency. The symbol "+" with the numeral refers to being better than that numeral, and the symbol "-" refers to being worse than that numeral. Polyester (PET) fabric or nylon (Ny) fabric were used for the evaluation.

| Water-repellency No. | Condition |
|---|---|
| 100: | Test fabric without wet and water droplets adhered on the surface. |
| 90: | Test fabric without wet but with small water droplets adhered on the surface. |
| 80: | Test fabric with wet in the form of separate small water droplets on the surface. |
| 70: | Test fabric with wet on half of the surface and in a state where separate small wet is penetrating the fabric. |
| 50: | Test fabric with wet on the entire surface. |
| 0: | Test fabric with wet on the entire surface and the entire back surface. |

### <Compound 2>

5.57 g of stearic acid (product of Tokyo Chemical Industry Co., Ltd.), 3.57 g of 2-amino 1,3-propanediol (product of FUJIFILM Wako Pure Chemical Corporation), 0.239 g of 4,4-dimethylaminopyridine (product of FUJIFILM Wako Pure Chemical Corporation), which were dissolved in 100.0 mL of dehydrated dimethylformamide (product of FUJIFILM Wako Pure Chemical Corporation), and 4.50 g of 1-(3-dimethylaminopropyl)-3-ethylcarbodiimide (product of Tokyo Chemical Industry Co., Ltd.), were added in this order, and the reaction solution was stirred at an oil bath temperature of 100°C overnight. The following day, the reaction solution was cooled to 40°C, filtered, and the solid was washed with dimethylformamide and water, and after washing, the solid was dried to yield 1.64 g of the compound 1 shown below.

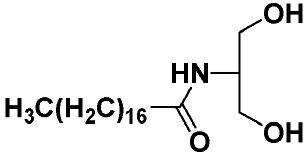

¹H NMR (CDCl3, 400 MHz) δ: 0.87 (t, 3H), 1.24-1.70 (m, 30H), 2.22 (t, 2H), 3.76-3.96 (m, 5H), 6.16 (brd, 2H)

1.64 g of the compound 1 which was dissolved in 35.0 mL of dehydrated tetrahydrofuran (product of FUJIFILM Wako Pure Chemical Corporation), and then 2.98 g of octadecyl isocyanate (product of FUJIFILM Wako Pure Chemical Corporation) and 4 drops of dibutyltin dilaurate were added in this order, and the reaction solution was stirred at an oil bath temperature of 70°C overnight. The following day, the reaction solution was cooled to room temperature, filtered, and the solid was washed with tetrahydofuran, and after washing, the solid was dried to yield 4.32 g of the compound 2 shown below.

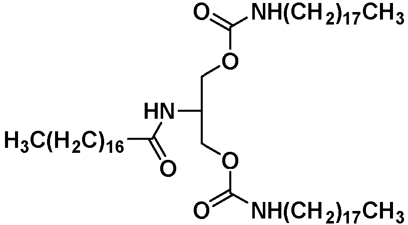

¹H NMR (CDCl3, 400 MHz) δ: 0.87 (t, 9H), 1.24-1.75 (m, 94H), 2.15 (t, 2H), 3.00-3.20 (m, 4H), 4.00-4.25 (m, 4H), 4.30-4.40 (m, 1H), 4.70-4.75 (m, 2H), 6.05 (brd, 2H)

### <Compound 3>

6.00 g of stearic acid (product of Tokyo Chemical Industry Co., Ltd.), 0.7 g of N,N'-di(2-aminoethyl)ethylenediamine (product of Tokyo Chemical Industry Co., Ltd.), 0.12 g of 4,4-dimethylaminopyridine (FUJIFILM Wako Pure Chemical Corporation), which were dissolved in 60.0 mL of dehydrated chloroform (FUJIFILM Wako Pure Chemical Corporation), and 4.13 g of 1-(3-dimethylaminopropyl)-3-ethylcarbodiimide (product of Tokyo Chemical Industry Co., Ltd.), were added in this order and the reaction solution was stirred at an oil bath temperature of 50°C overnight. The following day, the reaction solution was cooled to room temperature, filtered, and the solid was washed with methanol, diethyl ether and dichloromethane, and after washing, the solid was dried to yield 3.76 g of the compound 3 shown below.

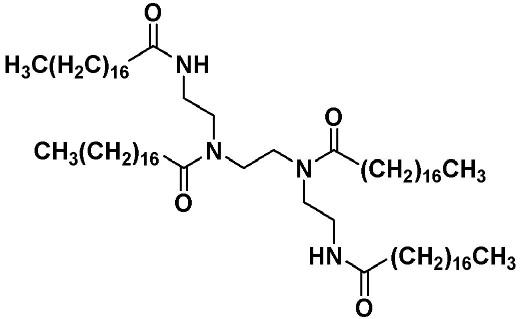

¹H NMR (CDCl3, 400 MHz) δ: 0.87 (t, 12H), 1.24-1.70 (m, 120H), 2.20-2.40 (m, 8H), 3.27-4.10 (m, 12H), 6.16 (brd, 2H)

### <Compound 4>

The compound 4 represented below was synthesized by referring to CN109280362A (2019-01-29).

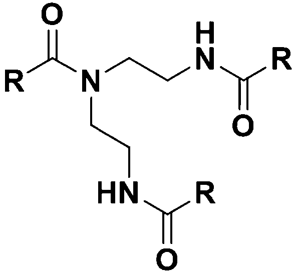

### [wherein R is a stearyl group.]

### <Examples 1 to 3>

Mold tests were carried out by using the compounds 2 to 4 obtained above.

2 g of the compound 2 was mixed with 0.2 g of polyoxyethylene (10) oleyl ether (HLB14), and the mixture was heated to 140°C and melted. The resulting solid was pulverized in a mortar, 17.8 g of water was added, and the mixture was then stirred in a homogenizer under the conditions of 10,000 rpm for 20 minutes to obtain a water dispersion composition of the compound 2. Subsequently, the water dispersion composition of the compound 2 was added to pulp slurry with a concentration of 0.5 wt% so that the ratio of the composition was 10 wt% in terms of solid content, relative to pulp, to prepare a pulp-containing aqueous composition. The pulp-containing aqueous composition was charged into an automatic mold molding machine to fabricate a pulp mold. The pulp mold underwent the practical oil-resistance Test at 65°C and the practical water-resistance test, which scored 4 points for both resistance.

2 g of the compound 3 was mixed with 0.2 g of polyoxyethylene (10) oleyl ether (HLB14), and the mixture was heated to 130°C and melted. The resulting solid was pulverized in a mortar, 17.8 g of water was added, and the mixture was then stirred in a homogenizer under the conditions of 10,000 rpm for 20 minutes to obtain a water dispersion composition of a compound 3. Subsequently, the water dispersion composition of the compound 3 was added to pulp slurry with a concentration of 0.5 wt% so that the ratio of the composition was 10 wt% in terms of solid content, relative to pulp, to prepare a pulp-containing aqueous composition. The pulp-containing aqueous composition was charged into an automatic mold molding machine to fabricate a pulp mold. The pulp mold underwent the practical oil-resistance Test at 65°C and the practical water-resistance test, which scored 4 points for both resistance.

2 g of the compound 4 was mixed with 0.2 g of polyoxyethylene (10) oleyl ether (HLB14), and the mixture was heated to 160°C and melted. The resulting solid was pulverized in a mortar, 17.8 g of water was added, and the mixture was then stirred in a homogenizer under the conditions of 10,000 rpm for 20 minutes to obtain a water dispersion composition of a compound 4. Subsequently, the water dispersion composition of the compound 4 was added to pulp slurry with a concentration of 0.5 wt% so that the ratio of the composition was 10 wt% in terms of solid content, relative to pulp, to prepare a pulp-containing aqueous composition. The pulp-containing aqueous composition was charged into an automatic mold molding machine to fabricate a pulp mold. The pulp mold underwent the practical oil-resistance Test at 65°C and the practical water-resistance test, which scored 4 points for both resistance.

The test results are summarized in Table 1 below. The melting points are also shown.

**[Table 1]**

| | | Oil-resistance and water-resistance (score) | | Melting point(°C) |
|---|---|---|---|---|
| | | Practical oil-resistance Test at 65°C | Practical water-resistance test | |
| Example 1 | Compound 2 | 4 | 4 | 105 |
| Example 2 | Compound 3 | 4 | 4 | 113 |
| Example 3 | Compound 4 | 4 | 4 | 143 |

### <Examples 4 and 5>

Using the compounds obtained above, the KIT test (oil resistance), the corn oil-resistance test, and the liquid repellency evaluation were conducted. The test results are summarized in Table 2 below.

**[Table 2]**

| | | Oil-resistance | | Liquid-repellency HD contact angle (static contact angle) (°) |
|---|---|---|---|---|
| | | KIT test (score) | Corn oil-resistance test | |
| Example 4 | Compound 3 | 6 | ○ | 45.3 |
| Example 5 | Compound 4 | 3 | ○ | 45.2 |

### <Example 6>

2 g of N,N'-ethylene bis-octadecanamide (biobased content: 97%, melting point: 143°C), 0.2 g of polyethylene glycol trimethylnonyl ether (HLB13), and 17.8 g of water were mixed to obtain a water dispersion composition (volume abundance ratio of particles with a size of 100 µm or larger: 3.9%, volume median diameter: 18 µm).

The water dispersion composition was added to pulp slurry with a concentration of 0.5 wt% so that the ratio of the composition was 3 wt% in terms of solid content, relative to pulp, to prepare a pulp-containing aqueous composition. The pulp-containing aqueous composition was charged into an automatic mold molding machine to fabricate a pulp mold.

The pulp mold underwent the practical oil-resistance Test at 110°C, the practical oil-resistance Test at 65°C and the practical water-resistance test, which scored 4 points for all the tests.

### <Example 7>

2 g of N,N'-ethylene bis oleic acid amide (biobased content: 96%, melting point: 116°C), 0.2 g of EO/PO polyalkylene glycol alcohol ether, and 17.8 g of water were mixed to obtain a water dispersion composition. (volume abundance ratio of particles with a size of 100 µm or larger: 0%, volume median diameter: 24 µm).

The water dispersion composition was added to pulp slurry with a concentration of 0.5 wt% so that the ratio of the composition was 10 wt% in terms of solid content, relative to pulp, to prepare a pulp-containing aqueous composition. The pulp-containing aqueous composition was charged into an automatic mold molding machine to fabricate a pulp mold.

The pulp mold underwent the practical oil-resistance Test at 65°C, which scored 4 points, and underwent the practical water-resistance test, which scored 4 points.

The KIT test (oil resistance) and corn oil-resistance test were conducted by using N,N'-ethylene bis oleic acid amide, resulting in a score of 4 points for the KIT test and evaluation as "o" for the corn oil-resistance test.

### <Example 8>

2 g of N,N'-xylylene-bis-12-hydroxystearylamide (biobased content: 83%, melting point: 124°C), 0.2 g of benzalkonium chloride, and 17.8 g of water were mixed to obtain a water dispersion composition (volume abundance ratio of particles with a size of 100 µm or larger: 0%, volume median diameter: 7 µm).

The water dispersion composition was added to pulp slurry with a concentration of 0.5 wt% so that the ratio of the composition was 3 wt% in terms of solid content, relative to pulp, to prepare a pulp-containing aqueous composition. The pulp-containing aqueous composition was charged into an automatic mold molding machine to fabricate a pulp mold.

The pulp mold underwent the practical oil-resistance Test at 65°C, which scored 4 points.

The KIT test (oil resistance) and corn oil-resistance test were conducted by using N,N'-xylylene-bis-12-hydroxystearylamide, resulting in a score of 3 points for the KIT test and evaluation as "o" for the corn oil-resistance test.

### <Example 9>

2 g of N,N'-xylylene-bis-12-hydroxystearylamide (biobased content: 83%), 0.2 g of polyethylene glycol trimethylnonyl ether (HLB13), and 17.8 g of water were mixed to obtain a water dispersion composition (volume abundance ratio of particles with a size of 100 µm or larger: 0%, volume median diameter: 9 µm).

The water dispersion composition was added to pulp slurry with a concentration of 0.5 wt% so that the ratio of the composition was 10 wt% in terms of solid content, relative to pulp, to prepare a pulp-containing aqueous composition. The pulp-containing aqueous composition was charged into an automatic mold molding machine to fabricate a pulp mold.

The pulp mold underwent the practical oil-resistance Test at 65°C, which scored 4 points, the pulp mold underwent the practical oil-resistance Test at 80°C, which scored 4 points, and underwent the practical oil-resistance Test at 120°C, which scored 4 points.

### <Example 10>

2 g of N,N'-xylylene-bis-12-hydroxystearylamide (biobased content: 83%), 0.2 g of potassium oleate, and 17.8 g of water were mixed to obtain a water dispersion composition (volume abundance ratio of particles with a size of 100 µm or larger: 0%, volume median diameter: 9 µm).

The water dispersion composition was added to pulp slurry with a concentration of 0.5 wt% so that the ratio of the composition was 10 wt% in terms of solid content, relative to pulp, to prepare a pulp-containing aqueous composition. The pulp-containing aqueous composition was charged into an automatic mold molding machine to fabricate a pulp mold.

The pulp mold underwent the practical oil-resistance Test at 65°C, which scored 4 points and underwent the practical water-resistance test, which scored 4 points.

### <Example 11>

2 g of N,N'-ethylene-bis-12-hydroxystearylamide (biobased content: 95%, melting point: 145°C), 0.2 g of EO/PO polyalkylene glycol natural alcohol ether, and 17.8 g of water were mixed to obtain a water dispersion composition (volume abundance ratio of particles with a size of 100 µm or larger: 5%, volume median diameter: 17 µm).

The water dispersion composition was added to pulp slurry with a concentration of 0.5 wt% so that the ratio of the composition was 5 wt% in terms of solid content, relative to pulp, to prepare a pulp-containing aqueous composition. The pulp-containing aqueous composition was charged into an automatic mold molding machine to fabricate a pulp mold.

The pulp mold underwent the practical oil-resistance Test at 65°C, which scored 4 points, and underwent the practical oil-resistance Test at 110°C, which scored 4 points.

### <Example 12>

2 g of N,N'-ethylene-bis-12-hydroxystearylamide (biobased content: 95%), 0.2 g of EO/PO polyalkylene glycol natural alcohol ether, and 17.8 g of water were mixed to obtain a water dispersion composition (volume abundance ratio of particles with a size of 100 µm or larger: 8.2%).

The water dispersion composition was added to pulp slurry with a concentration of 0.5 wt% so that the ratio of the composition was 10 wt% in terms of solid content, relative to pulp, to prepare a pulp-containing aqueous composition. The pulp-containing aqueous composition was charged into an automatic mold molding machine to fabricate a pulp mold.

The pulp mold underwent the practical oil-resistance Test at 80°C, which scored 4 points.

### <Example 13>

2 g of N,N'-ethylene-bis-12-hydroxystearylamide (biobased content: 95%), 0.2 g of EO/PO polyalkylene glycol natural alcohol ether, and 17.8 g of water were mixed to obtain a water dispersion composition (volume abundance ratio of particles with a size of 100 µm or larger: 5%, volume median diameter: 17 µm).

The water dispersion composition was added to pulp slurry with a concentration of 0.5 wt% so that the ratio of the composition was 3 wt% in terms of solid content, relative to pulp, to prepare a pulp-containing aqueous composition. The pulp-containing aqueous composition was charged into an automatic mold molding machine to fabricate a pulp mold.

The pulp mold underwent the practical water-resistance test, which scored 4 points.

### <Example 14>

2 g of N,N'-hexamethylene-bis-12-hydroxystearylamide (biobased content: 85%, melting point: 134°C), 0.2 g of EO/PO polyalkylene glycol natural alcohol ether, and 17.8 g of water were mixed to obtain a water dispersion composition (volume abundance ratio of particles with a size of 100 µm or larger: 3.5%, volume median diameter: 12 µm).

The water dispersion composition was added to pulp slurry with a concentration of 0.5 wt% so that the ratio of the composition was 7 wt% in terms of solid content, relative to pulp, to prepare a pulp-containing aqueous composition. The pulp-containing aqueous composition was charged into an automatic mold molding machine to fabricate a pulp mold.

The pulp mold underwent the practical oil-resistance Test at 65°C, which scored 4 points, the pulp mold underwent the practical oil-resistance Test at 80°C, which scored 4 points, and underwent the practical oil-resistance Test at 100°C, which scored 4 points.

### <Example 15>

2 g of the compound 3, 0.2 g of EO/PO polyalkylene glycol natural alcohol ether, and 17.8 g of water were mixed to obtain a water dispersion composition.

The water dispersion composition was added to pulp slurry with a concentration of 0.5 wt% so that the ratio of the composition was 10 wt% in terms of solid content, relative to pulp, to prepare a pulp-containing aqueous composition. The pulp-containing aqueous composition was charged into an automatic mold molding machine to fabricate a pulp mold.

### <Example 16>

2 g of the compound 5 represented by the formula:

N(CH₂CH₂NHCONHR)₃

[wherein R is a stearyl group.]
and 0.2 g of EO/PO polyalkylene glycol natural alcohol ether were mixed, the resulting solid was pulverized in a mortar and added with 17.8 g of water, and the mixture was then stirred in a homogenizer under the conditions of 10,000 rpm for 20 minutes to obtain a water dispersion composition of the compound 5 (volume abundance ratio of particles with a size of 100 µm or larger: 0%).

The water dispersion composition was added to pulp slurry with a concentration of 0.5 wt% so that the ratio of the composition was 7 wt% in terms of solid content, relative to pulp, to prepare a pulp-containing aqueous composition. The pulp-containing aqueous composition was charged into an automatic mold molding machine to fabricate a pulp mold.

The pulp mold underwent the practical oil-resistance Test at 65°C, which scored 4 points, the pulp mold underwent the practical oil-resistance Test at 80°C, which scored 4 points, and underwent the practical water-resistance test, which scored 4 points.

### <Example 17>

2 g of the compound 6 represented by the formula:

RNHCONHCH₂CH₂N(CONHR)CH₂CH₂N(CONHR)CH₂CH₂NHCONHR

[wherein R is a stearyl group.]
and 0.2 g of EO/PO polyalkylene glycol natural alcohol ether were mixed, the resulting solid was pulverized in a mortar and added with 17.8 g of water, and the mixture was then stirred in a homogenizer under the conditions of 10,000 rpm for 20 minutes to obtain a water dispersion composition of the compound 6 (volume abundance ratio of particles with a size of 100 µm or larger: 25.7%).

The water dispersion composition was added to pulp slurry with a concentration of 0.5 wt% so that the ratio of the composition was 10 wt% in terms of solid content, relative to pulp, to prepare a pulp-containing aqueous composition. The pulp-containing aqueous composition was charged into an automatic mold molding machine to fabricate a pulp mold.

The pulp mold underwent the practical oil-resistance Test at 65°C, which scored 5 points, the pulp mold underwent the practical oil-resistance Test at 80°C, which scored 5 points, and underwent the practical water-resistance test, which scored 5 points.

### <Example 18>

2 g of N,N'-ethylene bis-octadecanamide, 1.2 g of benzalkonium chloride, 0.8 g of polyethylene glycol trimethylnonyl ether, and 36 g of water were pulverized in a wet type micronization apparatus under the condition of 150 MPa to obtain a water dispersion composition (volume abundance ratio of particles with a size of 100 µm or larger: 0%, volume median diameter: 52 µm).

The water dispersion composition was added to pulp slurry with a concentration of 0.5 wt% so that the ratio of the composition was 10 wt% in terms of solid content, relative to pulp, to prepare a pulp-containing aqueous composition. The pulp-containing aqueous composition was charged into an automatic mold molding machine to fabricate a pulp mold.

The pulp mold underwent the practical oil-resistance Test at 65°C, which scored 4 points.

### <Example 19>

A biodegradability test of N,N'-ethylene bis oleic acid amide (biobased content: 96%, melting point: 116°C) was carried out according to JIS K 6953-1:2011. The biodegradability at a test period of 30 days was 65%, and the biodegradability at a test period of 60 days was 77%.

### <Example 20>

The measurement results of HD contact angles (n-hexadecane contact angles) of N,N'-ethylene-bis-octadecanamide, N,N'-ethylene-bis-oleic acid amide, N,N'-xylylene-bis-12-hydroxystearylamide, and N,N-ethylene-bis-12-hydroxystearylamide are shown in Table 3.

**[Table 3]**

| Liquid-repellent compound name | HD contact angle (n-hexadecane) | Water |
|---|---|---|
| | | Static contact angle/° |
| | Static contact angle/° | |
| N,N'-Ethylene bis-octadecanamide | 36.1 | 85.1 |
| N,N'-Ethylene bis oleic acid amide | 42.7 | 103.4 |
| N,N'-Xylylene-bis-12-hydroxystearylamide | 38.6 | 90.1 |
| N,N'-Ethylene-bis-12-hydroxystearylamide | 32.9 | 68.2 |

### <Paper Strength Improvement Effect of Pulp Mold by Addition of Repellent>

### <Example 21>

The evaluation results of the tensile strengths of the pulp mold fabricated in Example 6 (N,N'-ethylene bis-octadecanamide), the pulp mold fabricated in Example 8 (N,N'-xylylene-bis-12-hydroxystearylamide) and the pulp mold fabricated in Example 12 (N,N-ethylene-bis-12-hydroxystearylamide) are shown in Table 4.

### <Comparative Example 1>

Without having added the liquid-repellent compound, the pulp-containing aqueous composition was charged into an automatic mold molding machine to fabricate a pulp mold. The tensile strength of the pulp mold fabricated was evaluated, and the results are shown in Table 4.

**[Table 4. Evaluation Results of Tensile Test of Pulp Mold]**

| Evaluation example | Used pulp mold | Liquid-repellent compound name | Amount of liquid-repellent compound added (relative to pulp) | Tensile strength (N/mm²) |
|---|---|---|---|---|
| Evaluation 1 | Example 6 | N,N'-ethylenebisoctadecaneamide | 3 wt% | 18.92 |
| Evaluation 2 | Example 8 | N,N'-xylylene-bis-12-hydroxystearylamide | 3 wt% | 19.91 |
| Evaluation 3 | Example 12 | N,N'-ethylene-bis-12-hydroxystearylamide | 5 wt% | 19.25 |
| Evaluation 4 | Comparative Example 1 | Not added | - | 17.47 |

Table 4 shows that the liquid-repellent compound has effects of improving the tensile strength and paper strength.

### <Paper Strength Improvement Effect of Pulp Mold in Combination of Paper Strengthening Agent for Use>

### <Example 22>

The water dispersion composition of N,N'-xylylene-bis-12-hydroxystearylamide prepared in Example 8 was added to pulp slurry with a concentration of 0.5 wt% so that the ratio of the composition was 3 wt% in terms of solid content, relative to pulp, the mixture was stirred for 30 seconds, a polyacrylamide-based paper strengthening agent was then added in an amount of 0.1 wt% relative to pulp, and the mixture was stirred for 30 seconds to prepare a pulp-containing aqueous composition. The pulp-containing aqueous composition was charged into an automatic mold molding machine to fabricate a pulp mold. The pulp mold underwent the practical oil-resistance Test at 65°C, which scored 4 points. The evaluation results of the tensile strength of the fabricated pulp mold are shown in Table 5.

### <Example 23>

The water dispersion composition of N,N'-xylylene-bis-12-hydroxystearylamide prepared in Example 8 was added to pulp slurry with a concentration of 0.5 wt% so that the ratio of the composition was 3 wt% in terms of solid content, relative to pulp, the mixture was stirred for 30 seconds, a polyacrylamide-based paper strengthening agent was then added in an amount of 0.5 wt% relative to pulp, and the mixture was stirred for 30 seconds to prepare a pulp-containing aqueous composition. The pulp-containing aqueous composition was charged into an automatic mold molding machine to fabricate a pulp mold. The pulp mold underwent the practical oil-resistance Test at 65°C, which scored 4 points. The evaluation results of the tensile strength of the fabricated pulp mold are shown in Table 5.

### <Example 24>

The water dispersion composition of N,N'-xylylene-bis-12-hydroxystearylamide prepared in Example 8 was added to pulp slurry with a concentration of 0.5 wt% so that the ratio of the composition was 3 wt% in terms of solid content, relative to pulp, the mixture was stirred for 30 seconds, a polyacrylamide-based paper strengthening agent was then added in an amount of 1.1 wt% relative to pulp, and the mixture was stirred for 30 seconds to prepare a pulp-containing aqueous composition. The pulp-containing aqueous composition was charged into an automatic mold molding machine to fabricate a pulp mold. The pulp mold underwent the practical oil-resistance Test at 65°C, which scored 4 points. The evaluation results of the tensile strength of the fabricated pulp mold are shown in Table 5.

**[Table 5. Evaluation Results of Tensile Test of Pulp Mold]**

| Evaluation example | Liquid-repellent compound name | Amount of liquid-repellent compound added (relative to pulp) | Paper strengthening agent | Paper strengthening agent amount added (relative to pulp) | Tensile strength (N/mm²) |
|---|---|---|---|---|---|
| Example 22 | N,N'-xylylene-bis-12-hydroxystearylamide | 3 wt% | Polyacrylamide-based paper strengthening agent | 0.1 wt% | 22.2 |
| Example 23 | N,N'-xylylene-bis-12-hydroxystearylamide | 3 wt% | Polyacrylamide-based paper strengthening agent | 0.5 wt% | 28.2 |
| Example 24 | N,N'-xylylene-bis-12-hydroxystearylamide | 3 wt% | Polyacrylamide-based paper strengthening agent | 1.1 wt% | 27.1 |

### <Example 25>

The water dispersion composition of N,N'-xylylene-bis-12-hydroxystearylamide prepared in Example 8 was added to pulp slurry with a concentration of 0.5 wt% so that the ratio of the composition was 3 wt% in terms of solid content, relative to pulp, the mixture was stirred for 30 seconds, a cationic starch aqueous solution was the added in an amount of 3 wt% relative to pulp, and the mixture was stirred for 30 seconds to prepare a pulp-containing aqueous composition. The pulp-containing aqueous composition was charged into an automatic mold molding machine to fabricate a pulp mold. The pulp mold underwent the practical oil-resistance Test at 65°C, which scored 4 points.

### <Example 26>

The water dispersion composition of N,N'-xylylene-bis-12-hydroxystearylamide prepared in Example 8 was added to pulp slurry with a concentration of 0.5 wt% so that the ratio of the composition was 3 wt% in terms of solid content, relative to pulp, the mixture was stirred for 30 seconds, an alkyl ketene dimer dispersion was added in an amount of 0.5 wt% relative to pulp, and the mixture was stirred for 30 seconds to prepare a pulp-containing aqueous composition. The pulp-containing aqueous composition was charged into an automatic mold molding machine to fabricate a pulp mold. The pulp mold underwent the practical oil-resistance Test at 65°C, which scored 4 points.

### <Fabric Treatment: Water-Repellency Test> - reference test

### <Example 27>

### [Water-Repellency Test]

With use of N,N'-xylylene-bis-12-hydroxystearylamide, a treatment liquid with a solid concentration of 16.8 mg/mL was prepared, using chloroform as a solvent, PET fabric was immersed in this treatment liquid, passed through a mangle, and air-dried at room temperature for 24 hours. The fabric was then heat-treated at 140°C for 5 minutes and cooled to room temperature to prepare a test fabric. The test fabric was evaluated for water-repellency. The water-repellency test resulted in scoring 90 points.

### <Extraction of Compound from Pulp Mold and ¹H-NMR Measurement>

### <Example 28>

A pulp mold to which N,N'-ethylene bis-octadecanamide was added was fabricated in the same manner as in Example 6. Using a 3 mm-diameter punch, ten sections with a diameter of 3 mm were cut off from the bottom of the pulp mold. The 10 sections were placed in a 2-mL vial, 2 g of CDCl₃ was added therein, the vial was then covered with a lid and heated at 60°C for 1 hour. The CDCl₃ was transferred from the vial to an NMR tube and ¹H-NMR measurement was carried out to obtain the following results. The ¹H-NMR measurement demonstrated and confirmed the spectrum corresponding to N,N'-ethylenebisoctadecanamide. ¹H NMR (CDCl3, 400 MHz) δ: 0.88 (t, 6H), 1.20-1.80 (m, 60H), 2.17 (t, 2H), 2.35 (t, 1H), 3.39 (m, 2H)

### <Example 29>

A pulp mold added with N,N'-ethylene bis oleic acid amide was prepared in the same manner as in Example 7. ¹H-NMR measurement was carried out in the same manner as in Example 28, and the following results were obtained. The ¹H-NMR measurement confirmed the spectrum corresponding to N,N'-ethylene bis oleic acid amide.

¹H NMR (CDCl3, 400 MHz) δ: 0.88 (t, 6H), 1.20-1.80 (m, 44H), 1.90-2.21 (t, 12H), 3.38 (m, 4H), 5.28-5.42 (m, 4H)

### <Example 30>

A pulp mold added with N,N'-xylylene-bis-12-hydroxystearylamide was prepared in the same manner as in Example 9. ¹H-NMR measurement was carried out in the same manner as in Example 28, and the following results were obtained. The ¹H-NMR measurement confirmed the spectrum corresponding to N'-xylylene-bis-12-hydroxystearylamide. ¹H NMR (CDCl3, 400 MHz) δ: 0.88 (m, 6H), 1.20-1.80 (m, 64H), 2.21 (t, 3H), 4.43 (m, 4H), 7.15-7.21 (m, 4H)

## Claims

1. A use of a repellent for paper,
wherein the repellent comprises a liquid-repellent compound
wherein the liquid-repellent compound is a compound:
i) represented by the following formula:
N(-X-Rₙ)ₚ(-H)_{q}-L¹-[N(-X-Rₙ)ᵣ(-H)ₛ-L¹-]ₜ-N(-X-Rₙ)ₚ(-H)_{q}
wherein
X is a direct bond or a (1+n)-valent group, composed of one or more selected from the group consisting of a direct bond, -O-, -C(=O)-, -C(=NR'-), -S-, -S(=O)₂-, -NR'-, -C(OR')R'-, -C(OR')(-)₂, -N(-)₂, a divalent to tetravalent aliphatic hydrocarbon group having 1 to 10 carbon atoms, a divalent to tetravalent hydrocarbon aromatic ring, and a divalent to tetravalent heterocyclic ring, wherein R' is a hydrogen atom or a hydrocarbon group having 1 to 4 carbon atoms;
R each independently is a linear or branched monovalent C₆₋₄₀-hydrocarbon group optionally having a substituent;
L¹ is independently at each occurrence a divalent aliphatic hydrocarbon group or aromatic hydrocarbon group having 2 to 20 carbon atoms optionally interrupted by an oxygen atom and/or a sulfur atom;
n is independently at each occurrence an integer of 1 or more and 3 or less;
p is independently at each occurrence an integer of 0 or more and 2 or less;
q is independently at each occurrence an integer of 0 or more and 2 or less;
p + q is 2 in each N(-X-Rₙ)ₚ(-H)_{q};
r is independently at each occurrence 0 or 1;
s is independently at each occurrence 0 or 1;
r + s is 1 in each N(-X-Rₙ)ᵣ(-H)ₛ;
a sum of all p and all r is 1 or more; and
t is an integer of 0 or more and 10 or less;
or
ii) represented by the following formula:
N(-X-Rₙ)ₚ(-H)_{q}-L²(-X-Rₙ)ᵤ
wherein
X is a direct bond or a (1+n)-valent group, composed of one or more selected from the group consisting of a direct bond, -O-, -C(=O)-, -C(=NR'-), -S-, -S(=O)₂-, -NR'-, -C(OR')R'-, -C(OR')(-)₂, -N(-)₂, a divalent to tetravalent aliphatic hydrocarbon group having 1 to 10 carbon atoms, a divalent to tetravalent hydrocarbon aromatic ring, and a divalent to tetravalent heterocyclic ring, wherein R' is a hydrogen atom or a hydrocarbon group having 1 to 4 carbon atoms;
R each independently is a linear or branched monovalent C₆₋₄₀-hydrocarbon group optionally having a substituent;
L² is a 1 + u valent aliphatic hydrocarbon group or aromatic hydrocarbon group having 2 to 20 carbon atoms optionally interrupted by an oxygen atom and/or a sulfur atom;
n is independently at each occurrence an integer of 1 or more and 3 or less;
p is an integer of 0 or more and 2 or less;
q is an integer of 0 or more and 2 or less;
p + q is 2;
u is an integer of 1 or more and 3 or less;
a sum of p and u is 1 or more;
wherein the repellent is free of a fluorine compound.

2. The use of claim 1, wherein the liquid-repellent compounds apart from the (-X-Rₙ) groups are composed of a mono- to trivalent amino group and a chain saturated aliphatic hydrocarbon group or aromatic hydrocarbon group optionally interrupted by an O and/or S atom.

3. The use of claim 1 or 2, wherein the molar ratio of carbon to nitrogen atoms (C/N ratio) in the liquid-repellent compounds apart from the (-X-Rₙ) groups is ≤ 8.

4. The use of any of claims 1-3, wherein the liquid-repellent compound is a compound represented by the following formula: 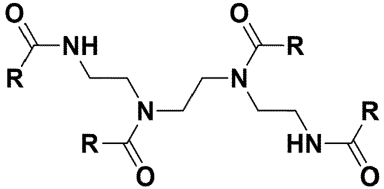 wherein
R is independently at each occurrence defined in the above, or
a compound represented by the following formula: 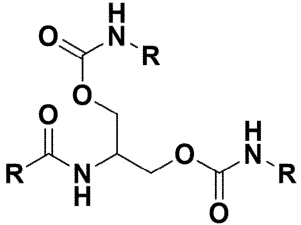
wherein
R is independently at each occurrence defined in the above.

5. The use of any of claims 1-4, wherein R has ≥ 12 carbon atoms.

6. The use of any of claims 1-5, wherein the divalent to tetravalent aliphatic hydrocarbon group having 1 to 10 carbon atoms has 5 or less carbon atoms.

7. The use of any of claims 1-6, wherein the liquid-repellent compound has
- a melting point of ≥ 40°C, or no melting point, and/or
- a n-hexadecane contact angle of ≥ 10°, and/or
- a biobased content of ≥ 30%.

8. The use of any of claims 1-7, wherein the volume abundance ratio of particles of the repellent with a size of ≥ 100 µm, measured by dynamic light scattering or laser diffraction scattering, is ≤ 25%.

9. The use of any of claims 1-8, wherein the repellent is a water dispersion.

10. The use of claim 9, wherein the repellent comprises one or more selected from a surfactant, silicone, wax, an organic acid, and a curing agent.

11. An article to which the liquid-repellent compound in the repellent defined in any of claims 1-10 is adhered; the article being oil-resistant or water-repellent paper, preferably a food packaging material or a food container.

12. A method for producing oil-resistant or water-repellent paper, comprising subjecting paper to external addition treatment or internal addition treatment with the repellent defined in any of claims 1-11.

## Patentansprüche

1. Verwendung eines Repellents für Papier,
wobei das Repellent eine flüssigkeitsabweisende Verbindung umfasst,
wobei die flüssigkeitsabweisende Verbindung eine Verbindung ist:
i) die durch die folgende Formel dargestellt wird:
N(-X-Rₙ)ₚ(-H)_{q}-L¹-[N(-X-Rₙ)ᵣ(-H)ₛ-L¹-]ₜ-N(-X-Rₙ)ₚ(-H)_{q}
wobei
X eine direkte Bindung oder eine (1+n)-wertige Gruppe ist, bestehend aus einer oder mehreren, ausgewählt aus der Gruppe, bestehend aus einer direkten Bindung, -O-,-C(=O)-, -C(=NR'-), -S-, -S(=O)₂-, -NR'-, -C(OR')R'-,-C(OR')(-)₂, -N(-)₂, einer zwei- bis vierwertigen aliphatischen Kohlenwasserstoffgruppe mit 1 bis 10 Kohlenstoffatomen, einem zwei- bis vierwertigen aromatischen Kohlenwasserstoffring, und einem zwei- bis vierwertigen heterocyclischen Ring, wobei R' ein Wasserstoffatom oder eine Kohlenwasserstoffgruppe mit 1 bis 4 Kohlenstoffatomen ist;
R jeweils unabhängig voneinander eine lineare oder verzweigte einwertige C₆₋₄₀-Kohlenwasserstoffgruppe ist, die gegebenenfalls einen Substituenten aufweist;
L¹ bei jedem Vorkommen unabhängig voneinander eine zweiwertige aliphatische Kohlenwasserstoffgruppe oder aromatische Kohlenwasserstoffgruppe mit 2 bis 20 Kohlenstoffatomen ist, die gegebenenfalls durch ein Sauerstoffatom und/oder ein Schwefelatom unterbrochen ist;
n bei jedem Vorkommen unabhängig voneinander eine ganze Zahl von 1 oder mehr und 3 oder weniger ist;
p ist bei jedem Vorkommen unabhängig voneinander eine ganze Zahl von 0 oder mehr und 2 oder weniger;
q ist bei jedem Vorkommen unabhängig voneinander eine ganze Zahl von 0 oder mehr und 2 oder weniger;
p + q ist in jedem N(-X-Rₙ)ₚ(-H)_{q} gleich 2;
r ist bei jedem Vorkommen unabhängig voneinander 0 oder 1;
s ist bei jedem Vorkommen unabhängig voneinander 0 oder 1;
r + s ist in jedem N(-X-Rₙ)ᵣ(-H)ₛ gleich 1;
die Summe aller p und aller r beträgt 1 oder mehr; und t ist eine ganze Zahl von 0 oder mehr und 10 oder weniger;
oder
ii) die durch die folgende Formel dargestellt wird:
N(-X-Rₙ)ₚ(-H)_{q}-L²(-X-Rₙ)ᵤ
wobei
X eine direkte Bindung oder eine (1+n)-wertige Gruppe ist, bestehend aus einer oder mehreren, ausgewählt aus der Gruppe, bestehend aus einer direkten Bindung, -O-,-C(=O)-, -C(=NR'-), -S-, -S(=O)₂-, -NR'-, -C(OR')R'-,-C(OR')(-)₂, -N(-)₂, einer zwei- bis vierwertigen aliphatischen Kohlenwasserstoffgruppe mit 1 bis 10 Kohlenstoffatomen, einem zwei- bis vierwertigen aromatischen Kohlenwasserstoffring, und einem zwei- bis vierwertigen heterocyclischen Ring, wobei R' ein Wasserstoffatom oder eine Kohlenwasserstoffgruppe mit 1 bis 4 Kohlenstoffatomen ist;
R jeweils unabhängig voneinander eine lineare oder verzweigte einwertige C₆₋₄₀-Kohlenwasserstoffgruppe ist, die gegebenenfalls einen Substituenten aufweist;
L² eine 1 + u-wertige aliphatische Kohlenwasserstoffgruppe oder aromatische Kohlenwasserstoffgruppe mit 2 bis 20 Kohlenstoffatomen ist, die gegebenenfalls durch ein Sauerstoffatom und/oder ein Schwefelatom unterbrochen ist;
n bei jedem Vorkommen unabhängig voneinander eine ganze Zahl von 1 oder mehr und 3 oder weniger ist;
p ist eine ganze Zahl von 0 oder mehr und 2 oder weniger;
q ist eine ganze Zahl von 0 oder mehr und 2 oder weniger;
p + q ist 2;
u ist eine ganze Zahl von 1 oder mehr und 3 oder weniger;
die Summe aus p und u ist 1 oder mehr;
wobei das Repellent frei von einer Fluorverbindung ist.

2. Verwendung gemäß Anspruch 1, wobei die flüssigkeitsabweisenden Verbindungen, abgesehen von den (-X-Rₙ)-Gruppen, aus einer ein- bis dreiwertigen Aminogruppe und einer kettengesättigten aliphatischen Kohlenwasserstoffgruppe oder aromatischen Kohlenwasserstoffgruppe bestehen, die gegebenenfalls durch ein O- und/oder S-Atom unterbrochen ist.

3. Verwendung gemäß Anspruch 1 oder 2, wobei das Molverhältnis von Kohlenstoff- zu Stickstoffatomen (C/N-Verhältnis) in den flüssigkeitsabweisenden Verbindungen, abgesehen von den (-X-Rₙ)-Gruppen, ≤ 8 beträgt.

4. Verwendung gemäß mindestens einem der Ansprüche 1 bis 3, wobei die flüssigkeitsabweisende Verbindung eine Verbindung ist, die durch die folgende Formel dargestellt wird: 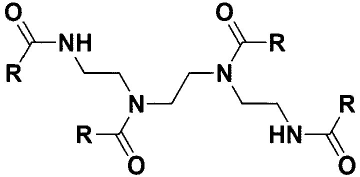 wobei
R bei jedem Vorkommen unabhängig voneinander wie oben definiert ist,
oder
eine Verbindung, die durch die folgende Formel dargestellt wird: 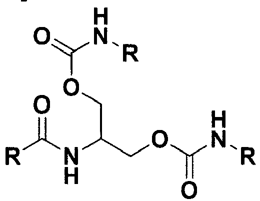
wobei
R bei jedem Vorkommen unabhängig voneinander wie oben definiert ist.

5. Verwendung gemäß mindestens einem der Ansprüche 1 bis 4, wobei R ≥ 12 Kohlenstoffatome aufweist.

6. Verwendung gemäß mindestens einem der Ansprüche 1 bis 5, wobei die zwei- bis vierwertige aliphatische Kohlenwasserstoffgruppe mit 1 bis 10 Kohlenstoffatomen 5 oder weniger Kohlenstoffatome aufweist.

7. Verwendung gemäß mindestens einem der Ansprüche 1 bis 6, wobei die flüssigkeitsabweisende Verbindung aufweist
- einen Schmelzpunkt von ≥ 40 °C, oder keinen Schmelzpunkt, und/oder
- einen n-Hexadecan-Kontaktwinkel von ≥ 10°, und/oder
- einen biobasierten Anteil von ≥ 30 %.

8. Verwendung gemäß mindestens einem der Ansprüche 1 bis 7, wobei das Volumenhäufigkeitsverhältnis von Partikeln des Repellents mit einer Größe von ≥ 100 µm, gemessen durch dynamische Lichtstreuung oder Laserdiffraktionsstreuung, ≤ 25 % beträgt.

9. Verwendung gemäß mindestens einem der Ansprüche 1 bis 8, wobei das Repellent eine Wasserdispersion ist.

10. Verwendung gemäß Anspruch 9, wobei das Repellent umfasst ein oder mehrere, ausgewählt aus einem Tensid, Silikon, Wachs, einer organische Säure, und einem Härtungsmittel.

11. Gegenstand, auf den die flüssigkeitsabweisende Verbindung in dem Repellent wie in einem der Ansprüche 1 bis 10 definiert aufgebracht ist; wobei es sich bei dem Gegenstand um ölbeständiges oder wasserabweisendes Papier handelt, vorzugsweise um ein Lebensmittelverpackungsmaterial oder einen Lebensmittelbehälter.

12. Verfahren zur Herstellung von ölbeständigem oder wasserabweisendem Papier, umfassend das Unterziehen des Papiers einer externen oder internen Additionsbehandlung mit dem Repellent wie in einem der Ansprüche 1 bis 11 definiert.

## Revendications

1. Utilisation d'un agent répulsif pour du papier,
dans laquelle l'agent répulsif comprend un composé répulsif pour les liquides
dans laquelle le composé répulsif pour les liquides est un composé :
i) représenté par la formule suivante :
N(-X-Rₙ)ₚ(-H)_{q}-L¹-[N(-X-Rₙ)ᵣ(-H)ₛ-L¹-]ₜ-N(-X-Rₙ)ₚ(-H)_{q}
dans lequel
X est une liaison directe ou un groupe (1+n)-valent, composé d'un ou plusieurs éléments sélectionnés dans le groupe consistant en une liaison directe, -O-, -C(=O), -C(=NR')-, -S-, -S(=O)₂-, -NR'-, -C(OR')R'-, -C(OR')(-)₂, -N(-)a, un groupe hydrocarboné aliphatique divalent à tétravalent présentant 1 à 10 atomes de carbone, un cycle aromatique hydrocarboné divalent à tétravalent et un cycle hétérocyclique divalent à tétravalent, où R' est un atome d'hydrogène ou un groupe hydrocarboné présentant 1 à 4 atomes de carbone ;
R est indépendamment à chaque occurrence un groupe hydrocarboné monovalent en C₆₋₄₀ linéaire ou ramifié présentant facultativement un substituant ;
L¹ est indépendamment à chaque occurrence un groupe hydrocarboné aliphatique divalent ou un groupe hydrocarboné aromatique présentant 2 à 20 atomes de carbone, interrompu facultativement par un atome d'oxygène et/ou un atome de soufre ;
n est indépendamment à chaque occurrence un nombre entier de 1 ou plus et inférieur ou égal à 3 ;
p est indépendamment à chaque occurrence un nombre entier de 0 ou plus et inférieur ou égal à 2 ;
q est indépendamment à chaque occurrence un nombre entier de 0 ou plus et inférieur ou égal à 2 ;
p + q est 2 dans chaque N(-X-Rₙ)ₚ(-H)_{q} ;
r est indépendamment à chaque occurrence 0 ou 1 ;
s est indépendamment à chaque occurrence 0 ou 1 ;
r + s est 1 dans chaque N(-X-Rₙ)ᵣ(-H)ₛ;
une somme de tous les p et de tous les r est 1 ou plus ; et
t est un nombre entier de 0 ou plus et inférieur ou égal à 10 ;
ou
ii) représenté par la formule suivante :
N(-X-Rₙ)ₚ(-H)q-L² (-X-Rₙ)u
dans lequel
X est une liaison directe ou un groupe (1+n)-valent, composé d'un ou plusieurs éléments sélectionnés dans le groupe consistant en une liaison directe, -O-, -C(=O), -C(=NR')-, -S-, -S(=O)₂-, -NR'-, -C(OR')R'-, -C(OR')(-)₂, -N(-)a, un groupe hydrocarboné aliphatique divalent à tétravalent présentant 1 à 10 atomes de carbone, un cycle aromatique hydrocarboné divalent à tétravalent, et un cycle hétérocyclique divalent à tétravalent, où R' est un atome d'hydrogène ou un groupe hydrocarboné présentant 1 à 4 atomes de carbone ;
R est indépendamment à chaque occurrence un groupe hydrocarboné monovalent en C₆₋₄₀ linéaire ou ramifié présentant facultativement un substituant ;
L² est un groupe hydrocarboné aliphatique ou un groupe hydrocarboné aromatique 1 + u valent présentant 2 à 20 atomes de carbone, interrompu facultativement par un atome d'oxygène et/ou un atome de soufre ;
n est indépendamment à chaque occurrence un nombre entier de 1 ou plus et inférieur ou égal à 3 ; p est un nombre entier de 0 ou plus et inférieur ou égal à 2 ; q est un nombre entier de 0 ou plus et inférieur ou égal à 2 ;
p + q est 2 ;
u est un nombre entier de 1 ou plus et inférieur ou égal à 3 ;
une somme de p et de u est de 1 ou plus ;
dans laquelle l'agent répulsif est exempt d'un composé fluoré.

2. Utilisation selon la revendication 1, dans laquelle les composés répulsifs pour les liquides, à part les groupes (-X-Rₙ), sont composés d'un groupe amino monovalent à trivalent et d'un groupe hydrocarboné aliphatique saturé en chaîne ou d'un groupe hydrocarboné aromatique facultativement interrompu par un atome d'oxygène et/ou un atome de soufre.

3. Utilisation selon la revendication 1 ou la revendication 2, dans laquelle le rapport molaire des atomes de carbone aux atomes d'azote (rapport C/N) dans les composés répulsifs pour les liquides à part les groupes (-X-Rₙ) est ≤ 8.

4. Utilisation selon l'une quelconque des revendications 1 à 3, dans laquelle le composé répulsif pour les liquides est un composé représenté par la formule suivante : 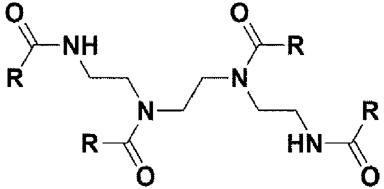 dans lequel
R est indépendamment à chaque occurrence tel que défini ci-dessus, ou
un composé représenté par la formule suivante : 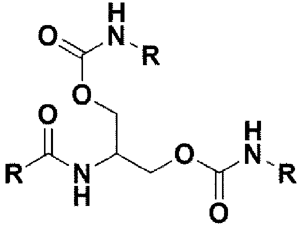
dans lequel
R est indépendamment à chaque occurrence tel que défini ci-dessus.

5. Utilisation selon l'une quelconque des revendications 1 à 4, dans laquelle R présente ≥ 12 atomes de carbone.

6. Utilisation selon l'une quelconque des revendications 1 à 5, dans laquelle le groupe hydrocarboné aliphatique divalent à tétravalent présentant 1 à 10 atomes de carbone présente un nombre d'atomes de carbone inférieur ou égal à 5.

7. Utilisation selon l'une quelconque des revendications 1 à 6, dans laquelle le composé répulsif pour les liquides présente
- un point de fusion de ≥ 40 °C ou aucun point de fusion, et/ou
- un angle de contact au n-hexadécane de ≥ 10°, et/ou
- une teneur biosourcée de ≥ 30 %.

8. Utilisation selon l'une quelconque des revendications 1-7, dans laquelle le rapport d'abondance volumique de particules de l'agent répulsif présentant une taille de ≥ 100 µm, mesuré par diffusion dynamique de la lumière ou diffusion par diffraction laser, est < 25 %.

9. Utilisation selon l'une quelconque des revendications 1-8, dans laquelle l'agent répulsif est une dispersion aqueuse.

10. Utilisation selon la revendication 9, dans laquelle l'agent répulsif comprend un ou plusieurs éléments sélectionnés parmi un tensioactif, du silicone, de la cire, un acide organique et un agent de durcissement.

11. Article auquel le composé répulsif pour les liquides dans l'agent répulsif défini dans l'une quelconque des revendications 1-10 est adhéré ; l'article étant du papier résistant à l'huile ou hydrofuge, de préférence un matériau d'emballage alimentaire ou un récipient alimentaire.

12. Procédé de production de papier résistant à l'huile ou hydrofuge, comprenant le fait de soumettre du papier à un traitement par addition externe ou à un traitement par addition interne avec l'agent répulsif défini dans l'une quelconque des revendications 1-11.
